(19) Europäisches Patentamt
European Patent Office
Office européen des brevets

(11) Publication number: 0 141 927 B1

(12) # EUROPEAN PATENT SPECIFICATION

(45) Date of publication of patent specification: 30.10.91

(51) Int. Cl.5: C07D 473/18, C07D 473/22, C07D 473/16, C07D 473/24, C07D 473/40, C07F 9/6561, A61K 31/52

(21) Application number: 84109539.1

(22) Date of filing: 10.08.84

The file contains technical information submitted after the application was filed and not included in this specification

(54) **Antiviral guanine derivatives.**

(30) Priority: 18.08.83 GB 8322199
21.09.83 GB 8325271
30.03.84 GB 8408322

(43) Date of publication of application: 22.05.85 Bulletin 85/21

(45) Publication of the grant of the patent: 30.10.91 Bulletin 91/44

(84) Designated Contracting States: BE CH DE FR GB IT LI NL SE

(56) References cited:

CHEMICAL ABSTRACTS, vol. 78, no. 9, 05 March 1973, Columbus, OH (US); U.K.PANDIT et al., p. 549, no. 58724n

(73) Proprietor: BEECHAM GROUP PLC
Beecham House Great West Road
Brentford Middlesex TW8 9BD(GB)

(72) Inventor: Jarvest, Richard Lewis
29 Beaconsfield Road
Surbiton Surrey(GB)
Inventor: Harnden, Michael Raymond
47 Guildford Road
Horsham Sussex(GB)

(74) Representative: Russell, Brian John
Beecham Pharmaceuticals Great Burgh Yew Tree Bottom Road
Epsom Surrey, KT18 5XQ(GB)

EP 0 141 927 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to compounds having antiviral activity, processes for their preparation and pharmaceutical compositions containing them.

EP-A-74306, EP-A-85424 and EP-A-72027 disclose guanine derivatives having antiviral activity. EP-A-152316 (only relevent for the purpose of Article 54(3)) discloses further guanine derivatives having antiviral activity.

The compound 9-(4-hydroxy-3-hydroxymethylbut-1-yl) guanine of formula (A)

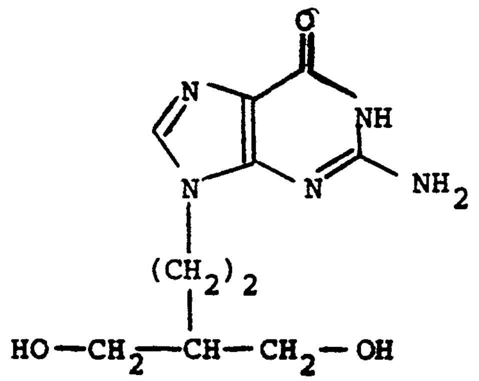

(A)

is disclosed in Synthetic Communications, 2(6), 345-351 (1972) but no pharmaceutical activity has been indicated for the compound in this or any other published document. We have repeated the synthesis of the compound as described in the above publication, and have shown that the product is a mixture of the compound of formula (A), its monobenzyl ether and its dibenzyl ether, this mixture having a melting point and uv spectrum in agreement with those reported in the publication for the supposedly 'pure' compound of formula (A). Our analysis of the product produced by the above synthesis showed that it contained 45-50% by weight of the compound of formula (A), 45-50% by weight of the monobenzyl ether and 5% or less by weight of the dibenzyl ether.

By different synthetic routes, we have prepared the compound of formula (A) in a substantially pure form and have found that it has anti-viral activity. This activity is also shown by certain derivatives of the compound of formula (A).

According to the present invention there is provided a compound of formula (I)

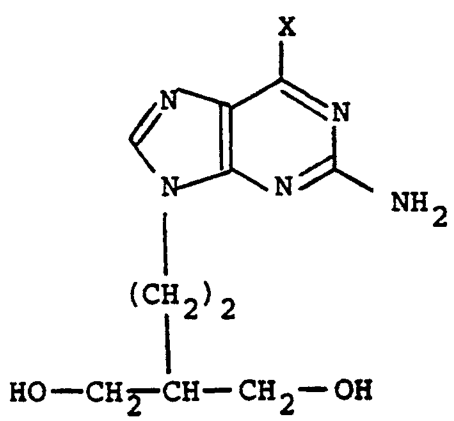

(I)

or a salt, phosphate ester or acyl derivative thereof (as hereinafter defined), in which X represents chlorine, straight or branched chain $C_{1-6}$ alkoxy, preferably methoxy, phenoxy, phenyl $C_{1-6}$ alkoxy, $-NH_2$, -OH or -SH with the proviso that, when X is -OH, the compound of formula (I) is in a purity state of greater than 50% by weight of pure compound.

The term 'acyl derivative' is used herein to include any derivative of the compounds of formula (I) in which one or more acyl groups are present. Such derivatives include biological precursors of the compounds of formula (I) in addition to those derivatives which are <u>per se</u> biologically active.

Acyl derivatives of the compounds of formula (I) are those wherein one or both of the hydrogens in the acyclic OH groups, and/or one of the hydrogen atoms in the $-NH_2$ group, are replaced by

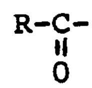

groups, wherein R is hydrogen or an alkyl, aryl, aralkyl or imidazolyl group.

Alkyl groups R are straight and branched chain groups containing up to 18 carbon atoms, preferably up to 6 carbon atoms. Particular examples are methyl, ethyl, t-butyl and pentyl.

An aryl group R is phenyl.

An aralkyl group R is a phenyl-$C_{1-6}$ alkyl group such as benzyl.

Examples of phosphate esters of the compounds of formula (I) include those where one or both of the acyclic -OH groups are replaced by

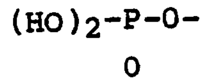

groups or salts thereof, or where the two -0H groups are replaced by a bridging

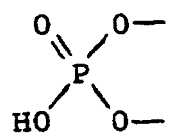

group

Salts, phosphate esters and acyl derivatives of the compounds of formula (I) are preferably pharmaceutically acceptable, but non-pharmaceutically acceptable compounds are also within the scope of the present invention, since these are useful as intermediates in the preparation of pharmaceutically acceptable compounds.

The compounds of formula (I) are defined herein as including tautomers of formula (I), wherein the -OH and -SH substituents are replaced by =0 and =S substituents respectively.

A particular group of compounds of the invention are those of formula (II)

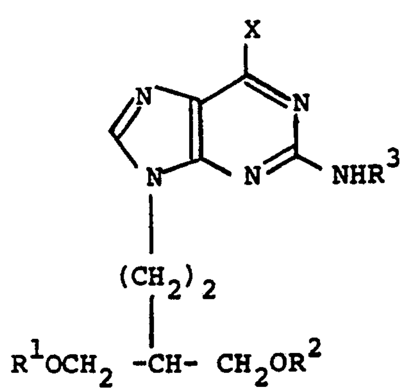

(II)

or pharmaceutically acceptable salts thereof, in which X is as defined in formula (I), and each of $R^1$, $R^2$ and $R^3$ represents hydrogen or an acyl group of formula

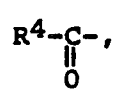

in which

$R^4$ is $C_{1-18}$ alkyl or imidazolyl, or $R^1$ or $R^2$ represents a phosphate ester group of formula

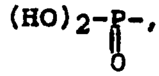

or $R^1$ and

$R^2$ together represent a

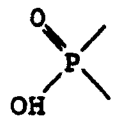

bridging group.

Subject to the aforementioned purity proviso in relation to compounds of the invention, a preferred compound of the present invention is the compound of formula (A)

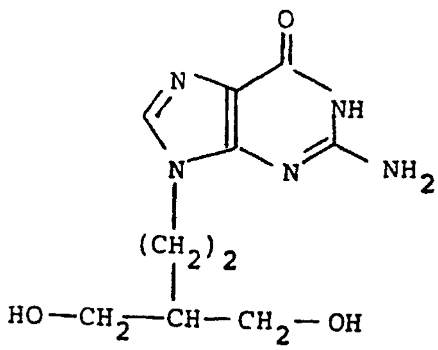

**(A)**

or a salt or acyl derivative therof.

In a further aspect of the invention there is provided a compound of formula (A) in a purity state of greater than 60% preferably greater than 80% more preferably greater than 90% and particularly preferably more than 95% by weight of pure compound.

In yet a further aspect of the invention, there is provided an isolated, substantially completely pure compound of formula (A), or a pharmaceutically acceptable salt thereof.

The invention also provides a compound of formula (A) in crystalline form having a melting point of 275-277°C.

The compounds of the present invention have antiviral activity, and are potentially useful in the treatment of infections caused by herpes viruses, such as herpes simplex type 1, herpes simplex type 2 and varicella zoster viruses.

Accordingly, the present invention also provides a compound of formula (I) or a pharmaceutically acceptable salt, phosphate ester or acyl derivative thereof, for use as an active therapeutic substance, and in particular for use in the treatment of viral infections. In this aspect of the invention, the compounds of formula (I) are not subject to the aforementioned purity proviso.

Examples of pharmaceutically acceptable salts of the compounds of formula (I) are those formed with organic bases, preferably with amines such as ethanolamines or diamines; and alkali metals, such as sodium and potassium; and acid addition salts formed with a pharmaceutically acceptable acid such as hydrochloric acid, orthophosphoric acid and sulphuric acid.

The compound of formula (A) or a salt thereof may be prepared by converting the group X in a compound of formula (III).

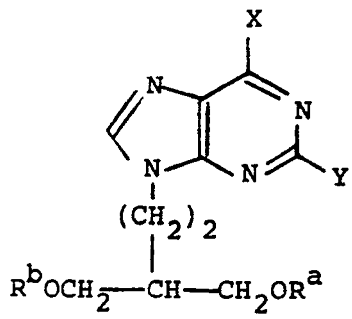

(III)

in which X, excluding - OH, is as defined in formula (I); $R^a$ and $R^b$, which may be the same or different, are each hydrogen or O- protecting groups, preferably acyl groups; and

Y is chlorine or $-NHR^c$, in which $R^c$ is hydrogen or acyl,

to an -OH group by means of hydrolysis, preferably acid hydrolysis, when X is other than $NH_2$, or, when X is $-NH_2$, by means of a deaminase reaction, or when Y is ohlorine and X is -OH, converting Y to a $-NH_2$ group by reaction with ammonia under pressure in accordance with known methods, and subsequently, if desired, converting the compound of formula (A) to a salt thereof by treatment with an acid or base.

Acyl groups $R^a$, $R^b$ and $R^c$ may be those of formula

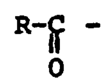

as hereinbefore defined.

Examples of groups $R^a$ and $R^b$ in formula (III) are acetyl and cyclic acetal such as isopropylidene. $R^c$ is preferably acetyl or hydrogen.

A preferred process for preparing the compound of formula (A) comprises treating a compound of formula (III) in which X is chlorine, Y is $-NH_2$ and $R^a$ and $R^b$ are each acetyl, with aqueous mineral acid, preferably hydrochloric acid.

Compounds of formulae (III) when each of $R^a$, $R^b$ and $R^c$ is hydrogen or acyl, are themselves compounds of the invention, having the additional utility as intermediates for the preparation of the compound of formula (A).

In a further aspect of the invention, compounds of formula (I) or acyl derivatives thereof, together with, compounds of formula (III), may be prepared by treating a compound of formula (IV).

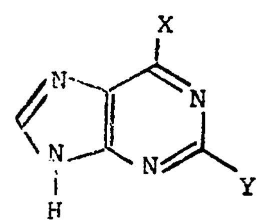

(IV)

in which X is as defined in formula (I) and Y is as defined in formula (III), with a compound of formula (V)

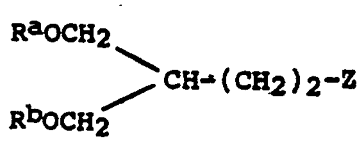

(V)

in which $R^a$ and $R^b$ are as defined in formula (III) and Z is a leaving group such as Cl, Br, or I, preferably Br.

Compounds of formula (IV) are either known compounds or can be made from known compounds by known methods.

Compounds of formula (V) in which Z is bromine may be prepared by brominating a compound of formula (VI).

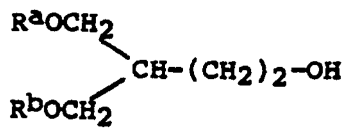 (VI)

preferably by treatment with carbon tetrabromide and triphenylphosphine in an organic, aprotic solvent, such as dimethylformamide.

Compounds of formula (V) in which Z is Cl or I may be prepared in an analogous manner.

Compounds of formula (VI) in which $R^a$ and $R^b$ are identical may be prepared according to the following schematic process.

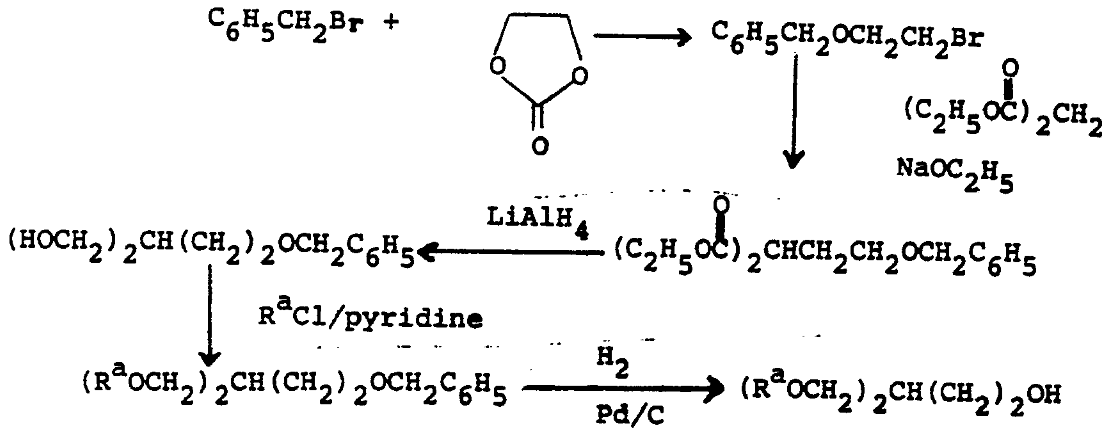


Acyl derivatives of compounds of formula (I) may also be prepared by acylating an optionally protected compound of formula (I) in accordance with conventional acylating processes known in the art, and where necessary, deprotecting the resulting product.

The acylation reaction may be carried out by using an acylating agent containing a

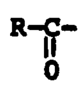

group, wherein R is as hereinbefore defined.

In a particular aspect of this process, the acylating agent contains the

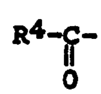

group, in which $R^4$ is $C_{1-18}$ alkyl, or is N,N'-carbonyldiimidazole.

Examples of acylating agents suitable for the above process are carboxylic acids, acid halides or acid anhydrides, preferably anhydrides or acids.

When the acylating agent is a carboxylic acid, a coupling agent such as dicyclohexylcarbodiimide should be included, but this is not necessary when the acylating agent is an acid anhydride.

The acylation reaction may produce a single acyl derivative of a compound of formula (I), or a mixture of derivatives, depending on a number of factors, such as the relative amounts and chemical natures of reactants, the physical conditions of the reaction, and the solvent system. Any mixture produced in this way may be separated into its pure components using standard chromatographic techniques.

The above described acylation process of the invention can yield mono-, di-, or tri-acylated derivatives of compounds of formula (I) according to the form of protection/deprotection utilised. The following are

examples of products obtained by different methods:

(a) Di-acylated derivatives of the two acyclic-OH groups may be obtained by direct acylation of unprotected compounds of formula (I) or acylation of protected intermediates of compounds of formula (I) in which the $-NH_2$ group is protected by, for example, a monomethoxytrityl group, and subsequent deprotection by treatment with acid.

(b) Mono-acylated derivatives of one of the acyclic -OH groups may be obtained by acylation of protected intermediates of compounds of formula (I) in which the $-NH_2$ group and the other acyclic -OH group are both protected by, for example, monomethoxytrityl groups, and subsequent deprotection by acid treatment.

(c) Mono-acylated derivatives of the $NH_2$ group may be obtained by acylation of protected intermediates of compounds of formula (I) in which both acyclic - OH groups are protected by, for example trimethylsilyl groups, and subsequent deprotection.

The various protected intermediates of compounds of formula (I) may be prepared in accordance with standard procedures by, for example, treatment of the compounds with monomethoxytrityl chloride (for processes (a) and (b)) or with chlorotrimethylsilane (for process (c)).

Protected intermediates of compounds of formula (I) may also be used to prepare phosphate esters of the compounds.

Accordingly, in a further process aspect of the invention, there is provided a process for preparing a mono-phosphate ester of a compound of formula (I) which comprises treating a protected intermediate of the compound of formula (I) in which one of the acyclic - OH groups and the $-NH_2$ group are protected, preferably by monomethoxytrityl groups, with cyano ethyl phosphoric acid and subsequently deprotecting the resultant product by treatment with acid, preferably acetic acid.

If desired, the reaction product after treatment with cyano ethyl phosphoric acid is treated with aqueous ammonia, which yields the ammonium salt of the phosphate ester as the final product.

Compounds of formula (I) or salts thereof may also be prepared by hydrolysing the 1,3-dioxane ring of a compound of formula (VII).

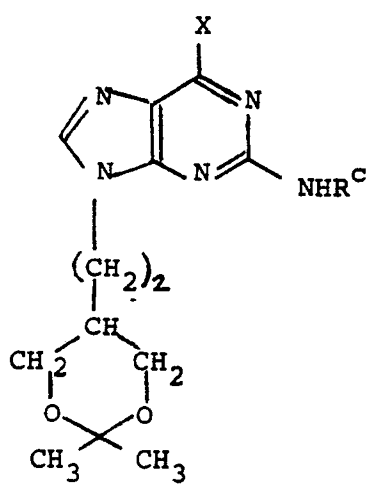

(VII)

in which X is as defined in formula (I) and $R^c$ is as defined in formula (III), provided that $R^c$ is not acyl when X is other than OH, and subsequently, if desired, converting the compound of formula (I) thus formed to a salt by treatment with an acid or base.

When $R^c$ is an acyl group, a basic N-deprotection step is required to form the compound of formula (A). This can be carried out prior to or after hydrolysis by treatment with, for example, (i) a solution of NaOMe in $CH_3OH$ or (ii) a solution of $NH_3$ in $CH_3OH$.

Preferably the hydrolysis of compounds of formula (VII) is carried out in acid medium. The compounds of formula (VII) in which X is alkoxy, phenoxy, phenylalkoxy or -SH are conveniently prepared <u>in situ</u> by reacting the compound of formula (VII) in which X is chlorine with an additional reactant containing an $X^1$ substituent, wherein $X^1$ is alkoxy, phenoxy, phenylalkoxy or sulphur. These intermediates can then be hydrolysed to compounds of formula (I) without isolating them from the reaction mixture.

The additional reactant containing the $X^1$ moiety may be a sodium alkoxide, phenoxide or phenylalkoxide, or sodium hydrosulphide (when $X^1$ is sulphur).

Acid hydrolysis of a compound of formula (VII) in which X is chlorine will yield a compound of formula

(I) in which X is chlorine, or a compound of formula (A) depending on acid strength and reaction conditions.

For example, treatment of the compound of formula (VII) in which X is chlorine with dilute HCl (1.OM) at 60°C for 24 hours or with 2 M HCl under reflux for 1.5 hours, will yield the compound of formula (A). Treatment of the same compound of formula (VII) with 2 M HCl in tetrahydrofuran at room temperature will yield the compound of formula (I) in which X is chlorine.

If desired, the compound of formula (VII) in which X is chlorine may be converted to the compound of formula (VII) in which X is amino, prior to acid hydrolysis.

The conversion may be achieved by treatment with sodium azide in dimethylformamide to form an azido intermediate in which X is replaced by an azide moiety, followed by reduction of the intermediate with ammonium formate/palladium-on-charcoal in methanol.

The intermediate compound of formula (VII) in which X is chlorine and $R^c$ is hydrogen may be prepared by treating a compound of formula (VIII).

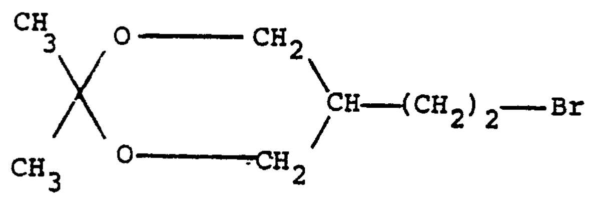

(VIII)

with a compound of formula (IX)

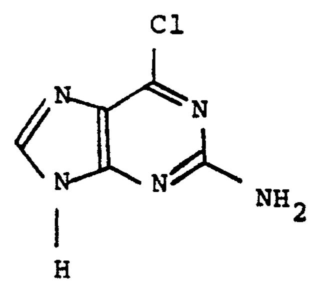

(IX)

The reaction may be carried out in an inert organic solvent, preferably dimethylformamide, in the presence of an inorganic base, preferably potassium carbonate.

The compound of formula (VIII) may itself be prepared by brominating a compound of formula (X)

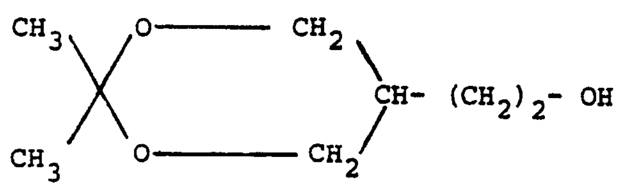

(X)

The reaction is preferably carried out by treating the compound of formula (X) with carbon tetrabromide and triphenylphosphine in an organic, aprotic solvent such as dimethylformamide.

The compound of formula (X) may itself be prepared by treating a compound of formula (XI)

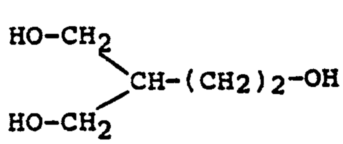

(XI)

with 2,2-dimethoxypropane and p-toluenesulphonic acid in the presence of acetone or tetrahydrofuran.

The compounds of formulae (IX) and (XI) are known compounds or can be prepared from known compounds by known methods.

The compounds of formulae (VII), (VIII) and (X) are novel intermediates and as such form further aspects of the present invention.

A compound of formula (I) or pharmaceutically acceptable salt, phosphate ester or acyl derivative thereof may be formulated for use in a pharmaceutical composition. Accordingly, in a further aspect of the invention, there is provided a pharmaceutical composition which comprises a compound of formula (I) or pharmaceutically acceptable salt, phosphate ester or acyl derivative thereof together with a pharmaceutically acceptable carrier or excipient.

A composition which may be administered by the oral route to humans may be compounded in the form of a syrup, tablet or capsule. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterile liquid carrier for injection.

The composition may also be formulated for topical application to the skin or eyes.

For topical application to the skin, the composition may be in the form of a cream, lotion or ointment. These formulations may be conventional formulations well known in the art, for example, as described in standard books of pharmaceutics and cosmetics, such as Harry's Cosmeticology published by Leonard Hill Books and the British Pharmacopaeia.

The composition for application to the eyes may be a conventional eye-drop composition well known in the art, or an ointment composition.

Preferably, the composition of this invention is in unit dosage form or in some other form that the patient may administer to himself a single dose. A suitable dosage unit might contain from 50 mg to 1 g of active ingredient, for example 100 to 500 mg. Such doses may be administered 1 to 4 times a day or more usually 2 or 3 times a day. The effective dose of compound will in general be in the range of from 1.0 to 20 mg/kg of body weight per day or more usually 2.0 to 10 mg/kg per day.

In a further aspect of the invention there is provided a compound of formula (I) or a pharmaceutically acceptable salt, phosphate ester or acyl derivative thereof for use as an active therapeutic substance, in particular for the treatment of viral infections in human or non-human animals.

The preparation of compounds of the invention is illustrated by the following Examples. Examples 4, 10-18, 21-27, 30-39 relate to compounds of formula (I) or salts, phosphate esters or acyl derivatives. The remaining examples relate to the preparation of intermediates.

Example 1

5-(2-Hydroxyethyl)-2,2-dimethyl-1,3-dioxan

To a suspension of lithium aluminium hydride (2.87g, 76mmol) in tetrahydrofuran (125ml), a solution of triethyl 1,1,2-ethanetricarboxylate (9.2ml, 9.85g, 40mmol) in tetrahydrofuran (25ml) was added dropwise with stirring over 2 hours. Excess reagent was then quenched with aqueous tetrahydrofuran (1:2). The inorganic salts were filtered off and washed with ethanol (100ml). The filtrate and washings were combined and the solvent was evaporated under reduced pressure to afford a colourless oil (4.85g). To a suspension of this oil in acetone (100ml), 2,2-dimethoxypropane (25ml) and p-toluenesulphonic acid monohydrate (2.3g, 12mmol) were added and the mixture was stirred for 1 hour. The resulting solution was neutralised with Amberlite *IR 45 ($OH^-$ form, methanol washed), filtered and the solvent evaporated under reduced pressure. The residue was purified by column chromatography on silica gel, eluting with chloroform-methanol mixtures(40:1 and 25:1) to afford 5-(2-hydroxyethyl)-2,2-dimethyl-1,3-dioxan as a colourless liquid (3.01g, 47%); $\nu$max (film) 3420, 2940, 1375, 1200 and 1080 $cm^{-1}$; $\delta_H$ ($CDCl_3$) 1.34-1.70 (8H, m, $C(CH_3)_2$ and $CH_2CH_2OH$), 1.7-2.1 (1H, m, CH), 2.15 (1H, br, $D_2O$ exchangeable, OH), and 3.5-4.0 (6H, m, 3 x $CH_2O$); (Found: C, 58.33; H, 10.11%. $C_8H_{16}O_3$ 0.25$H_2O$ requires C, 58.34; H, 10.10%. $[M-CH_3]^+$ found 145.0864; $C_7H_{13}O_3$ requires 145.0865).

Alternative Procedure for Preparation of 5-(2-Hydroxyethyl)-2,2-dimethyl-1,3-dioxan (Example 1)

A. 1,4-Dihydroxy-2-hydroxymethylbutane

*Trade Mark

To a refluxing solution of triethyl 1,1,2-ethanetricarboxylate (46ml, 200mmol) and sodium borohydride (20g, 530mmol) in t-butanol (400ml), methanol was added in 3 aliquots over 30 minutes (total 25ml). The solution was refluxed for a further 30 minutes and allowed to cool. Hydrochloric acid (5M) was carefully added to neutralise the solution. The solution was filtered and the inorganic residue was extracted with ethanol (2 x 100ml) and filtered. The organic solutions were combined and the solvent removed. The residue was extracted with ethanol (120ml) and the solution filtered. The solvent was removed to afford 1,4-dihydroxy-2-hydroxymethylbutane (24g, 100%); $\delta_H$ ($D_2O$)1.53 (2H, q, J 6Hz, 3-H), 1.75 (1H, m, 2-H), 3.57 (4H, d, J 6Hz, 1-H and 1'-H), and 3.64 (2H, t, J 6Hz, 4-H).

B. 5-(2-Hydroxyethyl)-2,2-dimethyl-1,3-dioxan

To a solution of 1,4-dihydroxy-2-hydroxymethylbutane (12g, 100mmol) in tetrahydrofuran (25ml), 2,2-dimethoxypropane (13.5g, 110mmol) and p-toluenesulphonic acid monohydrate (0.57g, 3mmol) were added. The solution was stirred for 0.5 hour at room temperature and was then neutralised by addition of triethylamine. The solvent was removed and the residue purified by column chromatography on silica gel eluting with chloroform-methanol mixtures to afford 5-(2-hydroxyethyl)-2,2-dimethyl-1,3-dioxan as a clear colourless liquid (6.5g, 41%).

Example 2

5-(2-Bromoethyl)-2,2-dimethyl-1,3-dioxan

To an ice-cooled solution of 5-(2-hydroxyethyl)-2,2-dimethyl-1,3-dioxan(1.92g, 12mmol) and carbon tetrabromide (7.96g, 24mmol) in dimethylformamide (100ml), triphenyl-phosphine (6.30g, 24mmol) was added and the solution was left at 4°C overnight. To this solution methanol (20ml) was added and the solvent was then evaporated under reduced pressure. The residue was purified by column chromatography on silica gel, eluting with hexane-acetone (12:1) to afford 5-(2-bromoethyl)-2,2-dimethyl-1,3-dioxan as a clear colourless liquid (0.89g, 40%); $\nu$max (film) 2940, 1370, 1270, 1260, 1200, and 1070 $cm^{-1}$; $\delta_H$ ($CDCl_3$) 1.42 (6H, s, $C(CH_3)_2$), 1.94 (3H, m, $CHCH_2CH_2Br$), 3.43 (2H, t, J 7Hz, $CH_2Br$), and 3.5-4.1 (4H, m, 2 x $CH_2O$); (Found: C, 42.84; H, 6.93 %. $C_8H_{15}BrO_2$ requires: C, 43.07; H, 6.78 %. $[M-CH_3]^+$ found 207.0024; $C_7H_{12}BrO_2$ requires 207.0021).

Alternative Procedure for Preparation of 5-(2-Bromoethyl)-2,2-dimethyl-1-3-dioxan (Example 2)

To an ice-cooled solution of 5-(2-hydroxyethyl)-2,2-dimethyl-1,3-dioxan(6.08g, 38mmol) and carbon tetrabromide (18.90g, 57mmol) in N,N-dimethylformamide (110ml), triphenyl-phosphine (14.95g, 57mmol) was added. The solution was stirred for 0.5 hour at 0°C. The solution was then diluted with saturated aqueous sodium bicarbonate (55ml) followed by water (55ml), and was extracted with hexane (2 x 150ml). The combined organic layers were dried (magnesium sulphate) and the solvent removed. The residue was placed under high vacuum for 2 hours to remove bromoform. The residue was taken up in a small amount of hexane, filtered and the solvent removed to afford 5-(2-bromoethyl)-2,2-dimethyl-1,3-dioxan (7.40g, 87%) as a colourless oil which crystallised on cooling, m.p. ca. 18°C.

Example 3

2-Amino-6-chloro-9-[2-(2,2-dimethyl-1,3-dioxan-5-yl)ethyl]-purine

To a solution of 5-(2-bromoethyl)-2,2-dimethyl-1,3-dioxan (0.75g, 3.7mmol) in dry dimethylformamide (12ml) 2-amino-6-chloropurine (0.68g, 4.0mmol) and then anhydrous potassium carbonate (0.83, 6.0mmol) were added. The solution was stirred at room temperature for 5 hours and left at 4°C overnight. The solution was filtered and the solvent removed. The residue was purified by column chromatography on silica gel, eluting with chloroform-methanol mixtures (80:1 and 60:1) to afford 2-amino-6-chloro-9-[2-(2,2-dimethyl-1,3-dioxan-5-yl)ethyl]purine as a white crystalline solid (0.74g, 64%), m.p. 125-126°C; $\lambda$max ($H_2O$) 223 ($\epsilon$ 28,900), 247 ($\epsilon$ 5,700), and 310 ($\epsilon$ 7,700) nm; $\nu$max (KBr) 3450, 3340, 1635, 1615, 1565, 1470, 1410, and 1375 $cm^{-1}$; $\delta_H$ [$(CD_3)_2SO$] 1.26 (3H, s, $CH_3$), 1.32 (3H, s, $CH_3$), 1.45-1.85 (3H, m, $CHCH_2CH_2N$), 3.51 (2H, dd, J 11Hz and J 7Hz, 2 x $H_{ax}$), 3.78 (2H, dd, J 11Hz and J 4Hz, 2 x $H_{eq}$), 4.05 (2H, t, J 7Hz, $CH_2N$), 6.89 (2H, s, $D_2O$ exchangeable, 2-$NH_2$), and 8.38 (1H, s, 8-H); (Found: C, 50.37; H, 5.68; N, 22.22 %; $M^+$ 311.1136. $C_{13}H_{18}ClN_5O_2$ requires C, 50.08; H, 5.82; N, 22.46 %; $M^+$ 311.1149).

Example 4

9-(4-Hydroxy-3-hydroxymethylbut-1-yl)guanine

2-Amino-6-chloro-9-[2-(2,2-dimethyl-1,3-dioxan-5-yl)-ethyl]purine (0.59g, 1.9mmol) in hydrochloric acid (1.0M, 4ml) was stirred at 60°C for 24 hours. The solution was diluted with water and neutralised with Amberlite * IR 45 ($OH^-$ form). The mixture was filtered, the resin washed with water and the solvent evaporated under reduced pressure. The residue was recrystallised from water to afford 9-(4-hydroxy-3-hydroxymethylbut-1-yl)guanine (238mg, 49%), m.p. 275-277°C; λmax ($H_2O$) 253 (ε 11,500) and 270 (shoulder, ε 8,630) nm; νmax (KBr) 3420, 3140, 1690, 1645, and 1605 $cm^{-1}$; $\delta_H$ [$(CD_3)_2SO$] 1.3-1.5 (3H, m, $CHCH_2CH_2$), 3.42 (4H, d, J 5Hz, 2 x $CH_2O$), 3.99 (2H, t, J 7Hz, $CH_2N$), 4.41 (2H, br, $D_2O$ exchangeable, 2 x OH), 6.44 (2H, s, $D_2O$ exchangeable, 2-$NH_2$), 7.71 (1H, s, 8-H), and 10.55 (1H, br, $D_2O$ exchangeable, 1-H); ($M^+$ found 253.1176. $C_{10}H_{15}N_5O_3$ requires $M^+$ 253.1175).

Alternative Procedure for Preparation of 9-(4-Hydroxy-3-hydroxymethylbut-1-yl)guanine (Example 4)

2-Amino-6-chloro-9-[2-(2,2-dimethyl-1,3-dioxan-5-yl)ethyl]purine (3.74g, 12mmol) in hydrochloric acid (2.0M, 12ml) was heated under reflux for 1.5 hours. The solution was neutralised with aqueous sodium hydroxide (10%) and then allowed to cool. The solution was filtered and the solid washed with water to afford 9-(4-hydroxy-3-hydroxymethylbut-1-yl)guanine as a white crystalline solid (2.18g, 72%), m.p. 275-277°C; (Found: C, 47.31; H, 6.02; N, 27.81 %; $C_{10}H_{15}N_5O_3$ requires C, 47.43; H, 5.97; N, 27.65 %).

Example 5

Ethyl 4-benzyloxy-2-ethoxycarbonylbutanoate

Sodium metal (72g, 3.13mmol) was dissolved in dry ethanol (1.21) with stirring, then diethyl malonate (477ml, 3.13mmol) and potassium iodide (249.5g, 1.5mmol) were added. An oil containing benzyl-2-bromoethyl ether (278.5g, 1.3mmol) contaminated with ethylene carbonate (198g) was then added slowly to the stirred mixture. On completion of the addition, the reaction mixture was heated under reflux for 18 hours, then poured into ice-water (7.51) and extracted with ether (3 x 1.61). The ether fractions were combined, dried ($MgSO_4$), filtered and evaporated to give an oil (550g). This was vacuum distilled to give ethyl 4-benzyloxy-2-ethoxycarbonylbutanoate (313g, 82%) as a clear oil, b.p. 165-180°/0.27kPa(2mm). $\delta_H$ - ($CDCl_3$) 1.22 (6H, t, 2 x $CH_3$), 2.18 (2H, q, $CHCH_2$), 3.48 (2H, t, $CHCH_2O$), 3.55 (1H, t, CH), 4.12 (4H, q, 2 x $CO_2CH_2$), 4.38 (2H, s, $OCH_2Ph$), 7.21 (5H, s, Ar).

Example 6

4-Benzyloxy-2-hydroxymethylbutan-1-ol

To a cooled, stirred suspension of lithium aluminium hydride (103g, 2.7mol) in dry ether (2.51) under nitrogen was added ethyl 4-benzyloxy-2-ethoxycarbonyl-butanoate (362g, 1.23mol) over a period of 3 hours. On completion of the addition, the reaction mixture was allowed to warm to room temperature, and then heated under reflux for 1 hour. It was then re-cooled and the excess lithium aluminium hydride destroyed by dropwise addition of water (100ml), 2M sodium hydroxide (100ml) and water (300ml). The reaction mixture was filtered, and the filter cake washed well with chloroform. The filtrate was dried ($MgSO_4$), filtered, and evaporated to give 4-benzyloxy-2-hydroxymethylbutan-1-ol as a clear oil (226g, 87%). $\delta_H$ ($CDCl_3$) 1.35-2.05 (3H, m, $CHCH_2CH_2$), 3.30-3.80 (8H, m, 3 x $CH_2O$, 2 x OH), 4.42 (2H, s, $OCH_2Ph$), 7.26 (5H, s, Ar).

Example 7

2-Acetoxymethyl-4-benzyloxybut-1-yl acetate

To a cooled solution of 4-benzyloxy-2-hydroxymethylbutan-1-ol in dry pyridine (1.1l) was added acetyl chloride (230ml, 3.24mol) over 2 hours, the temperature being maintained below 8°C. On completion of the addition, the reaction mixture was stirred at 5°C for a further 1 hour, then poured into water (41) and extracted with ethyl acetate (1 x 31, 1 x 21). The organic extracts were combined and washed with 2M hydrochloric acid (2 x 1l), water (1l) and brine (1l), dried ($MgSO_4$), filtered and evaporated to give a pale

*Trade Mark

yellow oil (300g).

Vacuum distillation afforded 2-acetoxymethyl-4-benzyloxybut-1-yl acetate as a colourless oil (220g, 70%) b.p. 160-165°/0.067kPa (0.05mm).

The fraction b.p. 122-160°/0.05mm (42g) was purified by column chromatography on silica gel, elution with ether-hexane 2:3 affording further 2-acetoxymethyl-4-benzyloxybut-1-yl acetate (27g, 8%). $\delta_H$ ($CDCl_3$) 1.68 (2H, q, $CHCH_2CH_2$), 2.01 (6H, s, 2 x $OCOCH_3$), 2.19 (1H, m, CH), 3.50 (2H, t, $CH_2OCH_2Ph$), 4.03 (4H, d, $CH_2OCOCH_3$), 4.43 (2H, s, $OCH_2Ph$), 7.24 (5H, s, Ar).

Example 8

2-Acetoxymethyl-4-hydroxybut-1-yl acetate

To a solution of 2-acetoxymethyl-4-benzyloxy-but-1-yl acetate (55g, 0.187mol) in ethanol (250ml) was added 10% palladium on carbon (2.5g), and the mixture hydrogenated at atmospheric pressure and room temperature. When the theoretical hydrogen uptake had been achieved (18 hours), the reaction was stopped and filtered through Celite * . Evaporation of the filtrate gave a colourless oil (35g). This was purified by column chromatography on silica gel, elution with 2% methanol in chloroform affording 2-acetoxymethyl-4-hydroxybut-1-yl acetate as a clear oil (32.9, 86%). $\delta_H$ ($CDCl_3$) 1.61 (2H, q, $CHCH_2CH_2$), 2.04 (6H, s, 2 x $OCOCH_3$), 2.20 (1H, m, CH), 2.61 (1H, br s, $D_2O$ exchangeable, OH), 3.68 (2H, t, $CH_2OH$), 4.04 (4H, d, 2 x $CH_2OCOCH_3$).

Example 9

2-Acetoxymethyl-4-bromobut-1-yl acetate

A mixture of 2-acetoxymethyl-4-hydroxy-but-1-yl acetate (10g, 49mmol), triphenyl phosphine (19.25g, 73mmol) and carbon tetrabromide (24.4g, 73mmol) was stirred for 18 hours at 4°C in dimethylformamide (150ml). The solvent was then evaporated, and the residue purified by column chromatography on silica gel, eluting with ether-light petroleum 2:3 to afford 2-acetoxymethyl-4-bromobut-1-yl acetate as a pale oil (130g, 99%). $\delta_H$ ($CDCl_3$) 1.73-2.56 (3H, m, $CHCH_2CH_2$), 2.04 (6H, s, 2 x $OCOCH_3$), 3.44 (2H, t, $CH_2Br$), 4.04 (4H, d, 2 x $CH_2OCOCH_3$).

Examples 11 and 10

9-(4-Acetoxy-3-acetoxymethylbut-1-yl)-2-amino-6-chloropurine and 7-(4-Acetoxy-3-acetoxymethylbut-1-yl)-2-amino-6-chloropurine

A mixture of 2-acetoxymethyl-4-bromobut-1-yl-acetate (13.0g, 48.7mmol), 2-amino-6-chloro-purine (8.25g, 48.7mmol) and anhydrous potassium carbonate (10g, 72.5mmol) was stirred in dry dimethylformamide (100ml) for 18 hours at room temperature. The reaction mixture was then filtered, the filtrate evaporated, and the residue purified by column chromatography on silica gel (500g). Elution with 3% methanol in chloroform afforded 9-(4-acetoxy-3-acetoxymethylbut-1-yl)-2-amino-6-chloropurine as a white solid (13.8g, 80%) mp 135-137°. $\lambda_{max}$ ($H_2O$) 222 ($\epsilon$ 28,500), 245 ($\epsilon$ 4,800) 307 ($\epsilon$ 7,700) nm; $\nu_{max}$ (KBr)3485, 3310, 3200, 1750, 1730, 1625, 1560, 1525, 1475, 1245 $cm^{-1}$. $\delta_H$ [$(CD_3)_2SO$] (270MHz) 1.85-2.05 (3H, m, $CHCH_2CH_2$), 2.01 (6H, s, 2 x $OCOCH_3$), 4.03 (4H, d, 2 x $CH_2OCOCH_3$), 4.16 (2H, t, $CH_2N$), 6.88 (2H, br s, $D_2O$ exchangeable, $NH_2$), 8.17 (1H, s, 8-H). Found C, 47.27; H, 4.94; N, 19.56%. $C_{14}H_{18}N_5O_4Cl$ requires C, 47.26; H, 5.10; N 19.68%.

Subsequent elution with 5% methanol in chloroform afforded 7-(4-acetoxy-3-acetoxymethylbut-1-yl)-2-amino-6-chloropurine as a white solid (2.9g, 17%) mp 174-175° (dec). $\lambda_{max}$ ($H_2O$) 222 ($\epsilon$ 25,000), 255 ($\epsilon$ 3,900), 318 ($\epsilon$ 5,600) nm; $\nu_{max}$ (KBr) 3390, 3310, 3205, 1745, 1735, 1635, 1550, 1505, 1380, 1365, 1310, 1250, 1240 $cm^{-1}$. $\delta_H$ [$(CD_3)_2SO$] (270MHz) 1.86 (2H, q, $CHCH_2CH_2$), 1.99 (6H, s, 2 x $OCOCH_3$), 1.95-2.05 (1H, m, CH), 4.03 (4H, d, 2 x $CH_2OCOCH_3$), 4.38 (2H, t, $CH_2N$), 6.60 (2H, br s, $D_2O$ exchangeable, $NH_2$), 8.39 (1H, s, 8-H). Found C, 47.48; H, 5.11; N, 19.52%. $C_{14}H_{18}N_5O_4Cl$ requires C, 47.26; H, 5.10; N, 19.68%.

Alternative procedure for preparation of 9-(4-Hydroxy-3-hydroxymethylbut-1-yl)guanine (Example 4)

*Trade Mark

A solution of 9-(4-acetoxy-3-acetoxymethyl-but-1-yl)-2-amino-6-chloropurine (15.5g, 43.6mmol) in 2M hydrochloric acid (150ml) was heated under reflux for 2 hours. The solution was then cooled to room temperature and neutralised with 10% sodium hydroxide solution, left to stand at 4°C, and the resulting precipitate filtered off, washed with cold water and recrystallized from water to give 9-(4-hydroxy-3-hydroxymethylbut-1-yl)guanine as a white crystalline solid (9.4g, 85%) mp 275-277°.

Example 12

9-(4-Hydroxy-3-hydroxymethylbut-1-yl)guanine, sodium salt

To a suspension of 9-(4-hydroxy-3-hydroxymethylbut-1-yl)guanine (0.30g, 1.2mmol) in water (8ml) was added aqueous sodium hydroxide (1 M, 1.2ml). The solvent was removed from the resulting clear solution and trituration with methanol-ethanol afforded 9-(4-hydroxy-3-hydroxymethylbut-1-yl)guanine sodium salt as a white solid (0.32g, 97%); λmax ($H_2O$, pH7.8) 252 (11,300) nm; νmax (KBr) 3400, 1590, and 1570 $cm^{-1}$; $\delta_H$ [$(CD_3)_2SO$] 1.47 (1H, m, 3'-H), 1.70 (2H, q, J 7Hz, 2'-H), 3.3-3.5 (4H, AB part of ABX, 2 x 4'-H), 3.96 (2H, t, J 7Hz, 1'-H), 4.7 (2H, br, $D_2O$ exchangeable, 2 x OH), 5.58 (2H, br.s, $D_2O$ exchangeable, 2-$NH_2$), and 7.43 (1H, s, 8-H).

Example 13

9-(4-Hydroxy-3-hydroxymethylbut-1-yl)guanine, potassium salt

To a suspension of 9-(4-hydroxy-3-hydroxymethylbut-1-yl)guanine (0.30g, 1.2mmol) in water (8ml) was added aqueous potassium hydroxide (1 M, 1.2ml). The solvent was removed from the resulting clear solution and trituration with methanol-ethanol afforded 9-(4-hydroxy-3-hydroxymethylbut-1-yl)guanine potassium salt as a white powdery solid (0.34g, 97%); λmax ($H_2O$, pH7.4) 252 (12,400); νmax (KBr) 3360, 3180, 1690, 1650, 1585, 1570, and 1480 $cm^{-1}$; $\delta_H$ [$(CD_3)_2SO$] 1.47 (1H, m, 3'-H), 1.69 (2H, q, J 7.1Hz,2'-H), 3.3-3.5 (4H, AB part of ABX, 2 x 4'-H), 3.94 (2H, t, J 7.3Hz, 1'-H), 4.7 (2H, br, $D_2O$ exchangeable, 2 x OH), 5.69 (2H, br.s, $D_2O$ exchangeable, 2-$NH_2$), and 7.38 (1H, s, 8-H); (Found: C, 41.11; H, 4.91; N, 23.82%; $C_{10}H_{14}N_5O_3K$ requires: C, 41.22; J, 4.84; N, 24.04%).

Example 14

2-Amino-6-chloro-9-(4-hydroxy-3-hydroxymethylbut-1-yl)purine hydrochloride

To a solution of 2-amino-6-chloro-9-[2-(2,2-dimethyl-1,3-dioxan-5-yl)ethyl]purine (0.46g, 1.5mmol) in tetrahydrofuran (4.5ml), hydrochloric acid (2.0M, 0.5ml) was added. A white precipitate formed and after 0.5 hour the solution was diluted with further tetrahydrofuran and was filtered to give 2-amino-6-chloro-9-(4-hydroxy-3-hydroxymethylbut-1-yl) purine hydrochloride (290mg, 63%), decomposed over 165°C; λmax ($H_2O$, pH5.5) 223 (ε 28,400), 245 (ε 4,620), and 307 (ε 7,620) nm; νmax (KBr) 3370, 3330, 3200, 2500, 1650, 1630, 1595, and 1505 $cm^{-1}$; $\delta_H$ [$(CD_3)_2SO$] 1.53 (1H, m, $CHCH_2CH_2$), 1.83 (2H, q, J 7Hz, $CHCH_2CH_2$), 3.35 (4H, d, J 6Hz, 2 x $CH_2O$), 4.19 (2H, t, J 7Hz, $CH_2N$), 5.85 (9H, s, $D_2O$ exchangeable, 2 x OH, $NH_2$, HCl and $H_2O$), and 8.57 (1H, s, 8-H). (Found: C, 39.04; H, 4.85; N, 22.36 %; $C_{10}H_{14}ClN_5O_4$.HCl requires C, 38.98; H, 4.91; N, 22.73 %).

Example 15

2-Amino-9-(4-hydroxy-3-hydroxymethylbut-1-yl)-6-methoxypurine

To a solution of 2-amino-6-chloro-9-[2-(2,2-dimethyl-1,3-dioxan-5-yl)ethyl]purine (0.28g, 0.9mmol) in methanol (2.5ml), methanolic sodium methoxide (1M, 1.0ml) was added and the solution was stirred at 50° for 1.5 hours. The solution was allowed to cool and hydrochloric acid (5M, 0.2ml) and water (0.4ml) were added. After 15 minutes the solution was neutralised with 10% aqueous sodium hydroxide. Silica gel was added and the solvent removed. Column chromatography on silica gel eluting with chloroform-methanol mixtures afforded 2-amino-9-(4-hydroxy-3-hydroxymethylbut-1-yl)-6-methoxypurine (185mg, 77%), m.p. 117-119°C; λmax ($H_2O$) 213 (ε 22,100), 249 (ε 6,860), and 280 (ε 8,410) nm; νmax (KBr) 3400, 3240, 3210, 1640, 1610, 1590, 1410, and 1395 $cm^{-1}$; $\delta_H$ [$(CD_3)_2SO$] 1.47 (1H, m, $CHCH_2CH_2$), 1.74 (2H, q, J 7Hz, $CHCH_2CH_2$), 3.40 (4H, d, J 6Hz, 2 x $CH_2O$), 3.95 (3H, s, $OCH_3$), 4.06 (2H, t, J 7Hz, $CH_2N$), 4.4 (2H, br, $D_2O$

exchangeable, 2 x OH), 6.33 (2H, s, $D_2O$ exchangeable, 2-$NH_2$), and 7.46 (1H, s, 8-H) (Found: $M^+$ 267.1340; $C_{11}H_{17}N_5O_3$ requires $M^+$ 267.1331).

Example 16

2-Amino-6-ethoxy-9-(4-hydroxy-3-hydroxymethylbut-1-yl)purine

To a suspension of 2-amino-6-chloro-9-[2-(2,2-dimethyl-1,3-dioxan-5-yl)ethyl]purine (0.31g, 1.0mmol) in ethanol (1.5ml) was added sodium ethoxide (1 M in ethanol, 1.5ml) and the mixture was stirred at 60° for 1 hour. The resulting solution was allowed to cool, hydrochloric acid (5 M, 0.3ml) and water (0.7ml) were added and the solution was stirred for 1 hour at room temperature. The solution was neutralised by addition of aqueous sodium bicarbonate and the solvent was removed. The residue was extracted with chloroform-ethanol (2:1), the solution was filtered and the solvent removed. The residue was purified by column chromatography on silica gel eluting with chloroform-methanol (6:1) to afford 2-amino-6-ethoxy-9-(4-hydroxy-3-hydroxymethylbut-1-yl)purine (0.24g, 85%), m.p. 150-152°C; λmax ($H_2O$) 213 (24,300), 249 (7,360), and 280 (9,270) nm; νmax (KBr) 3330, 3210, 2900, 1650, 1610, 1580 $cm^{-1}$; $\delta_H$ [$(CD_3)_2SO$] 1.3-1.6 (4H, m, 3'-H and $CH_3$), 1.73 (2H, q, J 7Hz, 2'-H), 3.2-3.6 (4H, AB part of ABX, 2 x 4'-H), 4.04 (2H, t, J 7Hz, 1'-H), 4.3-4.55 (4H, m, 2H $D_2O$ exchangeable, 2 x OH; $D_2O$ exchange leaves 2H, q, J 7Hz, 6-$OCH_2$), 6.30 (2H, s, $D_2O$ exchangeable, 2-$NH_2$), and 7.84 (1H, s, 8-H); (Found: C, 50.91; H, 7.00; N, 24.89%; $C_{12}H_{19}N_5O_3$ requires C, 51.23; H, 6.81; N, 24.90 %).

Example 17

2-Amino-6-benzyloxy-9-(4-hydroxy-3-hydroxymethylbut-1-yl) purine

A suspension of 2-amino-6-chloro-9-[2-(2,2-dimethyl-1,3-dioxan-5-yl)ethyl]purine (0.31g, 1.0mmol) in a solution of sodium benzoxide (1 M in benzyl alcohol, 2ml) was stirred at 70° for 1 hour. The resulting solution was allowed to cool, hydrochloric acid (5 M, 0.4ml) and water (0.6ml) were added and the solution was stirred for 1 hour at room temperature. The solution was then partitioned between chloroform and water. The aqueous layer was neutralised with aqueous sodium bicarbonate and extracted with chloroform. The combined organic layers were washed with aqueous sodium bicarbonate, dried (magnesium sulphate) and the solvent was removed. The residue was purified by column chromatography on silica gel eluting with chloroform-methanol mixtures (10:1, 5:1) to afford 2-amino-6-benzyloxy-9-(4-hydroxy-3-hydroxymethylbut-1-yl)purine as a white crystalline solid (0.17g, 50%), m.p. 146-147.5°C; λmax (EtOH) 212 (32,300), 250 (8,380), and 283 (10,100) nm; νmax (KBr) 3340, 3220, 1655, 1605, and 1580 $cm^{-1}$; $\delta_H$ [$(CD_3)_2SO$] 1.3-1.6 (1H, m, 3'-H), 1.72 (2H, q, J 7Hz, 2'-H), 3.38 (4H, AB part of ABX, 2 x 4'-H), 4.03 (2H, t, J 7Hz, 1'-H), 4.36 (2H, t, J 5.5Hz, $D_2O$ exchangeable, 2 x OH), 5.47 (2H, s, $PhCH_2$), 6.37 (2H, s, $D_2O$ exchangeable, 2-$NH_2$), 7.3-7.6 (5H, m, $C_6H_5$), and 7.84 (1H, s, 8-H) ; (Found: C, 58.89; H, 6.12; N, 19.87%; $C_{17}H_{21}N_5O_3$ requires C, 59.46; H, 6.16; N, 20.40%).

Example 18

2-Amino-9-(4-hydroxy-3-hydroxymethylbut-1-yl)-6-thiopurine

A solution of 2-amino-6-chloro-9-[2-(2,2-dimethyl-1,3-dioxan-5-yl)ethyl]purine (0.31g, 1.0mmol) in aqueous sodium hydrosulphide (2M, 3.0ml) and ethanol (1.5ml) was stirred at 70°C for 1 hour. To this solution glacial acetic acid (2.5ml) was added and the mixture was stirred for a further 1 hour at 70°C. The solution was allowed to cool, filtered and the solvent removed. The residue was recrystallised from water to afford 2-amino-9-(4-hydroxy-3-hydroxymethylbut-1-yl)-6-thiopurine (0.13g, 48%), m.p. decomposed at 260°C; $\delta_H$ [$(CD_3)_2SO$] 1.45 (1H, m, $CHCH_2CH_2$), 1.72 (2H, q, J 7Hz, $CHCH_2CH_2$), 3.3-3.5 (4H, ABX $J_{AB}$ 10.7Hz, $J_{AX}$ 5.5Hz and $J_{BX}$ 5.8Hz, 2 x $CH_2O$), 4.02 (2H, t, J 7.4Hz, $CH_2N$), 4.45 (2H, br, $D_2O$ exchangeable, 2 x OH), 6.77 (2H, s, $D_2O$ exchangeable, 2-$NH_2$), 7.87 (1H, S, 8-H), and 11.9 (1H, br, $D_2O$ exchangeable, 1-H); λmax ($H_2O$) 230 (ε 16,900), 263 (ε 7,210) and 341 (ε 25,200) nm; νvmax (KBr) 3310, 3130, 1650, 1610, and 1580 $cm^{-1}$; (Found: C, 44.86; H, 5.60; N, 25.44 %; $C_{10}H_{15}N_5O_2S$ requires C, 44.60; H, 5.61; N, 26.00 %).

Example 19

2-Amino-6-azido-9-[2-(2,2-dimethyl-1,3-dioxan-5-yl)ethyl]purine

To a solution of 2-amino-6-chloro-9-[2-(2,2-dimethyl-1, 3-dioxan-5-yl)ethyl]purine (0.47g, 1.5mmol) in dry N,N-dimethylformamide (5ml), sodium azide (0.20g, 3.0mmol) was added and the mixture was stirred at 100-110°C for 4 hours. The solvent was removed and the residue washed with water to leave 2-amino-6-azido-9-[2-(2,2-dimethyl-1,3-dioxan-5-yl)ethyl]purine as a crystalline solid (0.36g, 75%), m.p. decomposed at 200°C; λmax (MeOH) 272 (ε 8,210) and 301 (ε 10,100) nm; νmax (KBr) 1670, 1625, and 1560 $cm^{-1}$; $\delta_H$ - ($CDCl_3$-$CD_3OD$) 1.44 (6H, S, $C(CH_3)_2$), 1.6-2.2 (3H, m, $CHCH_2CH_2$), 3.5-3.8 (2H, dd (ABX), J 7Hz and J 11Hz,2 x $H_{ax}$), 3.85-4.15 (2H, dd (ABX), J 4Hz and J 11Hz, 2 x $H_{eq}$), 4.29 (2H, t, J 7Hz, $CH_2N$), and 7.93 (1H, s, 8-H) (Found: C, 48.96; H, 5.66; N, 35.15 %; $M^+$ 318.1553. $C_{13}H_{18}N_8O_2$ requires C, 49.05; H, 5.70; N, 35.20 %; $M^+$ 318.1546).

Example 20

2,6-Diamino-9-[2-(2,2-dimethyl-1,3-dioxan-5-yl)ethyl]purine

A mixture of 2-amino-6-azido-9-[2-(2,2-dimethyl-1,3-dioxan-5-yl)ethyl]purine (318mg, 1.0mmol), formic acid (0.15ml, 4.0mmol), concentrated ammonia (0.22ml, 4.0mmol), 10% palladium-on-charcoal (30mg) and methanol (10ml) was heated under reflux for 1 hour. The solution was allowed to cool, filtered and the solvent removed. The residue was purified by column chromatography on silica gel eluting with chloroform-methanol mixtures (20:1 and 15:1) to give 2,6-diamino-9-[2-(2,2-dimethyl-1,3-dioxan-5-yl)ethyl]purine (190mg, 65%), m.p. 202-204°C); νmax (KBr) 1670, 1640, 1595, and 1410 $cm^{-1}$; $\delta_H$ ($CDCl_3$-$CD_3OD$) 1.42 (6H, s, $C(CH_3)_2$), 1.6-2.0 (3H, m, $CHCH_2CH_2$), 3.5-4.2 (6H, m, 2 x $CH_2O$ and $CH_2N$), and 7.68 (1H, s, 8-H) (Found: C, 52.88; H, 6.78; N, 28.36 %; $M^+$ 292.1652. $C_{13}H_{20}N_6O_2$ requires C, 53.41; H, 6.90; N, 28.75 %; $M^+$ 292.1648).

Example 21

2,6-Diamino-9-(4-hydroxy-3-hydroxymethylbut-1-yl)purine

A solution of 2,6-diamino-9-[2-(2,2-dimethyl-1,3-dioxan-5-yl)ethyl]purine (180mg, 0.6mmol) in 70% acetic acid (10ml) was stirred for 1 hour at room temperature. The solvent was removed, the residue was suspended in methanol and sodium methoxide was added to neutralise. Column chromatography on silica gel eluting with chloroform-methanol mixtures (6:1, 4:1 and 3:1) gave 2,6-diamino-9-(4-hydroxy-3-hydroxymethylbut-1-yl)purine (95mg, 63%), m.p. 187-190°C; λmax ($H_2O$, pH6.5) 215 (ε 25,500), 255 (ε 7,290), and 280 (ε 9,170) nm; νmax 3150, 1680, 1650, 1605, 1590, and 1410 $cm^{-1}$; $\delta_H$ [$(CD_3)_2SO$] 1.46 (1H, m, $CHCH_2CH_2$),1.73 (2H, q, J 7.1Hz, $CHCH_2CH_2$), 3.3-3.5 (4H, ddd (ABX), $J_{AB}$ 10.6Hz, $J_{AX}$ 5.5Hz and $J_{BX}$ 5.9Hz, 2 x $CH_2O$), 4.01 (2H, t, J 7.3Hz, $CH_2N$), 4.41 (2H, br, $D_2O$ exchangeable, 2 x OH), 5.70 (2H, S, $D_2O$ exchangeable, $NH_2$), 6.56 (2H, S, $D_2O$ exchangeable, $NH_2$), and 7.69 (1H, s, 8-H) (Found: C, 46.09; H, 6.32; N, 31.69 %; $C_{10}H_{16}N_6O_2.0.1CHCl_3$ requires C, 45.91; H, 6.14; N, 31.81 %).

Example 22

9-(4-Acetoxy-3-acetoxymethylbut-1-yl)guanine

A mixture of 9-(4-hydroxy-3-hydroxymethylbut-1-yl)guanine (253mg, 1.0mmol), 4-dimethylaminopyridine (25mg) and acetic anhydride (8.5ml) was stirred for 4 days at room temperature. The acetic anhydride was removed under reduced pressure. The residue was purified by column chromatography on silica gel eluting with chloroform-methanol mixtures (20:1 and 10:1) to afford 9-(4-acetoxy-3-acetoxymethylbut-1-yl)guanine (160mg, 47%) which was recrystallised from methanol, m.p. 202-205°C; νmax (KBr) 1737, 1690, 1628, 1600, and 1240 $cm^{-1}$; $\delta_H$ [$(CD_3)_2SO$] 1.79 (2H, q, J 6.7Hz, $CHCH_2CH_2$), 1.91 (1H, m, $CHCH_2CH_2$), 2.00 (6H, s, 2 x $CH_3$), 4.00 (6H, m, 2 x $CH_2O$ and $CH_2N$), 6.42 (2H, s, $D_2O$ exchangeable, 2-$NH_2$), 7.71 (1H, s, 8-H), and 10.54 (1H, s, $D_2O$ exchangeable, 1-H); $\delta_C$ [$(CD_3)_2SO$] 20.71 (2 x $CH_3$), 28.29 (C-2'), 34.54 (C-3'), 40.59 (C-1'), 63.58 (2 x C-4'), 116.64 (C-5), 137.58 (C-8), 151.24 (C-4), 153.38 (C-2), 156.87 (C-6), and 170.57 (2 x COO) (Found: C, 49.62; H, 5.70; N, 20.51 %; $M^+$ 337.1392; $C_{14}H_{19}N_5O_5$ requires C, 49.85; H, 5.68; N, 20.76 %; $M^+$ 337.1386).

Examples 23 and 24

9-(4-Propionyloxy-3-propionyloxymethylbut-1-yl)guanine (Example 23) and $N^2$-Propionyl-9-(4-propionyloxy-

3-propionyloxymethylbut-1-yl)guanine (Example 24)

A mixture of 9-(4-hydroxy-3-hydroxymethylbut-1-yl)guanine (253mg, 1.0mmol), 4-dimethylaminopyridine (30mg), propionic anhydride (8ml) and N,N-dimethylformamide (15ml) was stirred at room temperature for 66 hours. The solvent was removed and the residue subjected to column chromatography on silica gel eluting with chloroform-methanol mixtures (30:1, 20:1, 10:1). The first compound to elute was $N^2$-propionyl-9-(4-propionyloxy-3-propionyloxymethylbut-1-yl)guanine (200mg, 47%) which was recrystallised from ether-methanol, m.p. 152-154°C; $\nu$max (KBr) 1740, 1675, 1610, 1560, and 1185 $cm^{-1}$; $\delta_H$ ($CDCl_3$) 1.14 (6H, t, J 7.5Hz, 2 x $OCOCH_2CH_3$), 1.27 (3H, t, J 7.5Hz, $NCOCH_2CH_3$), 1.88 (2H, q, J 6.9Hz, $CHCH_2CH_2$), 2.01 (1H, m, $CHCH_2CH_2$), 2.35 (2H, q, J 7.5Hz, 2 x $OCOCH_2CH_3$), 2.56 (2H, q, J 7.5Hz, $NCOCH_2CH_3$), 4.1-4.3 (6H, m, 2 x $CH_2O$ and $CH_2N$), 7.65 (1H, s, 8-H), 9.14 (1H, s, N-H), and 11.95 (1H, s, N-H) (Found: C, 54.13; H, 6.44; N, 16.19 %; $M^+$ 421.1958; $C_{19}H_{27}N_5O_6$ requires C, 54.15; H, 6.46; N, 16.62 %; $M^+$ 421.1961).

The second compound to elute was 9-(4-propionyloxy-3-propionyloxymethylbut-1-yl)guanine (150mg, 41%) which was recrystallised from methanol, m.p. 204-206°C; $\nu$max (KBr) 3310, 3150, 1740, 1690, and 1190 $cm^{-1}$; $\delta_H$ [$(CD_3)_2SO$] 1.01 (6H, t, J 7.4Hz, 2 x $CH_2CH_3$), 1.80 (2H, q, J 7.0Hz, $CHCH_2CH_2$), 1.91 (1H, m, $CHCH_2CH_2$), 2.29 (4H, q, J 7.5Hz, 2 x $CH_2CH_3$), 4.01 (6H, m, 2 x $CH_2O$ and $CH_2N$), 6.37 (2H, S, $D_2O$ exchangeable, 2-$NH_2$), 7.69 (1H, S, 8-H), and 10.50 (1H, s, $D_2O$ exchangeable, 1-H) (Found: C, 52.28; H, 6.20; N, 18.95 %; $C_{16}H_{23}N_5O_5$ requires C, 52.59; H, 6.35; N, 19.17 %).

Example 25

9-(4-Hexanoyloxy-3-hexanoyloxymethylbut-1-yl)guanine

A mixture of 9-(4-hydroxy-3-hydroxymethylbut-1-yl)guanine (253mg, 1.0mmol), dicyclohexylcarbodiimide (0.83mg, 4.0mmol), hexanoic acid (0.38ml, 0.35g, 3.0mmol), 4-dimethylaminopyridine (20mg) and N,N-dimethylformamide (5ml) was stirred for 64 hours at room temperature. The mixture was diluted with water and extracted with chloroform (x 2). The combined organic layers were washed with aqueous sodium bicarbonate, dried (magnesium sulphate) and the solvent removed. The residue was purified by column chromatography eluting with chloroform-methanol mixtures to afford 9-(4-hexanoyloxy-3-hexanoyloxymethylbut-1-yl)guanine(200mg, 45%) which was recrystallised from methanol, m.p. 198.5-201°C; $\nu$max (KBr) 3340, 3160, 2960, 2930, 1740, 1690, 1650, 1605, and 1170 $cm^{-1}$; $\delta_H$ ($CDCl_3$) 0.87 (6H, t, J 6.9Hz, 2 x $CH_3$), 1.28 (8H, m, 2 x $CH_2CH_2CH_3$), 1.60 (4H, quintet, J 7.4Hz, 2 x $COCH_2CH_2$), 1.90 (2H, q, J 6.9Hz, $CHCH_2CH_2N$), 2.02 (1H, m, $CHCH_2CH_2N$), 2.30 (4H, t, J 7.6Hz, 2 x $COCH_2CH_2$), 4.13 (6H, m, 2 x $CH_2O$ and $CH_2N$), 6.42 (2H, s, $D_2O$ exchangeable, 2-$NH_2$), 7.70 (1H, s, 8-H), and 12.16 (1H, s, $D_2O$ exchangeable, 1-H) (Found: C, 58.97; H, 7.92; N, 15.45 %; $C_{22}H_{35}N_5O_5$ requires C, 58.78; H, 7.85; N, 15.58 %).

Example 26

9-(4-Formyloxy-3-formyloxymethylbut-1-yl)guanine

A mixture of 9-(4-hydroxy-3-hydroxymethylbut-1-yl)guanine (0.23g, 0.9mmol), dicyclohexylcarbodiimide (0.92g, 4.5mmol), formic acid (0.17ml, 4.5mmol), 4-dimethylamino-pyridine (20mg) and N,N-dimethylformamide (5ml) was stirred for 40 minutes at room temperature and then quenched by addition of methanol (1ml). The solution was filtered and the solvent removed. The residue was purified by column chromatography on silica gel eluting with chloroform-methanol mixtures (7:1, 4:1) to afford 9-(4-formyloxy-3-formyloxymethylbut-1-yl)guanine which was crystallised from methanol (0.12g, 43%), m.p. 195-198°C; $\nu$max (KBr) 1720, 1680, 1630, 1600, and 1570 $cm^{-1}$; $\delta_H$ [$(CD_3)_2SO$] 1.83 (2H, q, J 7.1Hz, 2'-H), 2.01 (1H, m, 3'-H), 4.04 (2H, t, J 7.1Hz, 1'-H), 4.13 (4H, d, J 5.5Hz, 2 x 4'-H), 6.39 (2H, s, $D_2O$ exchangeable, 2-$NH_2$), 7.70 (1H, s, 8-H), 8.23 (2H, s, 2 x HCOO), and 10.52 (1H, s, $D_2O$ exchangeable, 1-H); (Found: C, 45.40; H, 4.68; N, 21.70%; $C_{12}H_{15}N_5O_5$ requires: C, 46.60; H, 4.89; N, 22.64%).

Example 27

9-[4-(N-Imidazolylcarbonyloxy)-3-(N-imidazolylcarbonyloxymethyl)but-1-yl]guanine

A mixture of 9-(4-hydroxy-3-hydroxymethylbut-1-yl)guanine (253mg, 1.0mmol), N,N'-carbonyldiimidazole (187mg, 1.15mmol), 4-dimethylaminopyridine (20mg) and N,N-dimethylformamide (5ml) was

stirred at room temperature. After 2 hours a further quantity of N,N'-carbonyldiimidazole (180mg) was added and stirring was continued for a further 2 hours. The solvent was removed and the residue washed with water ethyl acetate and hot methanol leaving 9-[4-(N-imidazolylcarbonyloxy)-3-(N-imidazolylcarbonyloxymethyl)but-1-yl]guanine (360mg, 82%), m.p. >300°C; νmax (KBr) 3320, 3140, 1760, 1695, 1630, and 1600 cm$^{-1}$; $\delta_H$ [$(CD_3)_2SO$] 2.0 (2H, q, J 7Hz, $CHCH_2CH_2$),2.15-2.40 (1H, m, $CHCH_2CH_2$), 4.10 (2H, t, J 7Hz, $CH_2N$), 4.48 (4H, d, J 5Hz, 2 x $CH_2O$), 6.32 (1H, s, $D_2O$ exchangeable, 2-$NH_2$), 7.05 (2H, s, imid-H), 7.57 (2H, s, imid-H), 7.75 (1H, s, 8-H), 8.28 (2H, s, imid-H), and 10.53 (1H, s, $D_2O$ exchangeable, 1-H); M/Z 68 (100, imidazole$^+$), 44 (70, $CO_2^+$), 41 (72, $NCHN^+$).

Example 28 & 29

$N^2$-Monomethoxytrityl-9-(4-monomethoxytrityloxy-3-hydroxymethylbut-1-yl)guanine
and
$N^2$- Monomethoxytrityl -9-(4-hydroxy-3-hydroxymethylbut-1-yl)guanine

A solution of 9-(4-hydroxy-3-hydroxymethylbut-1-yl)guanine (4.05g, 16mmol), monomethoxytrityl chloride (10.9g 35mmol), triethylamine (6.7ml) and 4-dimethylaminopyridine (40mg) in N,N-dimethylformamide (50ml) was stirred for 2 hours. The reaction was quenched with methanol and the solvent was removed. The residue was taken up in ethyl acetate and the solution washed with aqueous sodium bicarbonate and water. The solution was dried (magnesium sulphate) and the solvent removed. The residue was purified by column chromatography on silica gel eluting with chloroform-methanol mixtures. The first major product to elute was $N^2$-monomethoxytrityl-9-(4-monomethoxytrityloxy-3-hydroxymethylbut-1-yl)guanine (4.4g, 34%), m.p. 142-145°C; λmax (EtOH) 230 (sh. 29,900) and 262 (16,000) nm; νmax (KBr) 3400, 1680, 1605, 1570, and 1510 cm$^{-1}$; $\delta_H$ [$(CD_3)_2SO$] 1.24 (2H, m, 2'-H), 1.43 (1H, m, 3'-H), 2.7-2.9 (2H, AB part of ABX, $CH_2OC$), 3.1-3.4 (2H, AB part of ABX, $CH_2OH$), 3.42 (2H, t, J 6.7Hz, 1'-H), 3.66 (3H, s, $CH_3O$), 3.74 (3H, s, $CH_3O$), 4.35 (1H, t, J 4.8Hz, $D_2O$ exchangeable, OH), 6.7-7.4 (28H, m, Ar-H), 7.44 (1H, s, 8-H), 7.55 (1H, s, $D_2O$ exchangeable, 2-NH), and 10.50 (1H, s, $D_2O$ exchangeable, 1-H); (Found: C, 74.28; H, 5.86; N, 8.64%; $C_{50}H_{47}N_5O_5$ requires: C, 75.26; H, 5.94; N, 8.78%).

The second major product to elute was $N^2$-monomethoxytrityl-9-(4-hydroxy-3-hydroxymethylbut-1-yl)-guanine (1.4g, 17%), m.p. 205-207°C; λmax (EtOH) 261 (14,500) nm; νmax (KBr) 3380, 1705, 1680, 1610, 1570, and 1515 cm$^{-1}$; $\delta_H$ [$(CD_3)_2SO$] 1.25 (3H, m, 2'-H and 3'-H), 3.1-3.3 (4H, m, 2 x 4'-H), 3.52 (2H, t, J 6.6Hz, 1'-H), 3.72 (3H, s, $CH_3O$), 4.28 (2H, t, J 5.2Hz, $D_2O$ exchangeable, 2 x OH), 6.85-7.35 (14H, m, Ar-H), 7.54 (1H, s, 8-H), 7.56 (1H, s, $D_2O$ exchangeable, 2-NH), and 10.49 (1H, s, $D_2O$ exchangeable, 1-H); (Found: C, 67.93; H, 6.05; N, 12.90%; $C_{30}H_{31}N_5O_4$ requires C, 68.55; H, 5.95; N, 13.32%).

Example 30

9-(4-Pivalyloxy-3-pivalyloxymethylbut-1-yl)guanine

To a solution of $N^2$-monomethoxytrityl-9-(4-hydroxy-3-hydroxymethylbut-1-yl)guanine (0.47g, 0.9mmol) in pyridine (4.5ml) was added pivalyl chloride (0.55ml, 4.5mmol) and the solution was stirred for 45 minutes. The mixture was precipitated in water (45ml) and the resulting precipitate was stirred in 80% acetic acid (10ml) at 80° for 20 minutes. The solvent was removed and the residue was purified by column chromatography on silica gel eluting with chloroform-methanol (10:1) to afford 9-(4-pivalyloxy-3-pivalyloxymethylbut-1-yl)guanine (0.22g, 58%), m.p. 225-237°C; νmax (KBr) 3430, 2980, 1730, 1690, and 1620 cm$^{-1}$; $\delta_H$ [$(CD_3)_2SO$] 1.11 (18H, s, 2 x $C(CH_3)_3$), 1.81 (2H, q, J 6.8HZ, 2'-H), 1.93 (1H, m, 3'-H), 4.0-4.1 (6H, m, 1'-H and 2 x 4'-H), 6.38 (2H, s, 2-$NH_2$), 7.69 (1H, s, 8-H), and 10.58 (1H, br.s, 1-H); (Found: C, 56.58; H, 7.26; N, 16.14%; $C_{20}H_{31}N_5O_5$ requires: C, 56.99; H, 7.41; N, 16.62%).

Example 31

9-(4-Acetoxy-3-hydroxymethylbut-1-yl)guanine

To a solution of $N^2$-monomethoxytrityl-9-(4-monomethoxytrityloxy-3-hydroxymethylbut-1-yl)guanine (0.72g, 0.9mmol) in pyridine (3ml) was added acetyl chloride (0.21ml, 3.0mmol) and the solution was stirred for 30 minutes. The mixture was precipitated in water (30ml) the resulting precipitate was stirred in 80% acetic acid (10ml) at 80° for 30 minutes. The solvent was removed and the residue purified by column chromatography on silica gel eluting with chloroform-methanol mixtures (7:1, 3:1) to afford 9-(4-acetoxy-3-hydroxymethylbut-1-yl)guanine (0.17g, 64%), m.p. 194-200°C; νmax (KBr) 3330, 3170, 2930, 1730, 1690, 1660, 1610, and 1565 $cm^{-1}$; $\delta_H$ [$(CD_3)_2SO$] 1.6-1.8 (3H, m, 2'-H and 3'-H), 1.98 (3H, s, $CH_3$), 3.39 (2H, br, $D_2O$ exchange gives d, J 5Hz, $CH_2OH$), 3.9-4.1 (4H, m, 1'-H and $CH_2OCO$), 4.61 (1H, br.t, $D_2O$ exchangeable, OH), 6.44 (2H, s, $D_2O$ exchangeable, 2-$NH_2$), 7.68 (1H, s, 8-H), and 10.59 (1H, s, $D_2O$ exchangeable, 1-H); (Found: C, 47.91; H, 5.63; N, 21.71%; $C_{12}H_{17}N_5O_4$ requires: C, 48.81; H, 5.80; N, 23.72%).

Example 32

9-(4-Benzoyloxy-3-hydroxymethylbut-1-yl)guanine

To a solution of $N^2$-monomethoxytrityl-9-(4-monomethoxytrityloxy-3-hydroxymethylbut-1-yl)guanine (0.72g, 0.9mmol) in pyridine (4ml) was added benzoyl chloride (0.31ml, 2.7mmol) and the solution was stirred for 30 minutes. The mixture was precipitated in water (40ml) and the resulting precipitate was stirred in 80% acetic acid (10ml) at 80° for 45 minutes. The solvent was removed and the residue purified by column chromatography on silica gel eluting with chloroform-methanol mixtures (6:1, 3:1) to afford 9-(4-benzoyloxy-3-hydroxymethylbut-1-yl)guanine (80mg, 25%), m.p. 156-168°C; λmax (MeOH) 231 (15,100) and 254 (13,100) nm; νmax (KBr) 1715, 1690, 1625, and 1600 $cm^{-1}$; $\delta_H$ [$(CD_3)_2SO$] 1.75-1.90 (3H, m, 2'-H and 3'-H), 3.50 (2H, t, J 5Hz, $D_2O$ exchange leaves d, $CH_2OH$), 4.07 (2H, t, J 6.9Hz, 1'-H), 4.2-4.35 (2H, AB part of ABX, $CH_2OCO$), 4.68 (1H, t, J 5.1Hz, $D_2O$ exchangeable, OH), 6.39 (2H, s, $D_2O$ exchangeable, 2-$NH_2$), 7.5-8.0 (6H, m, $C_6H_5$ and 8-H), and 10.53 (1H, s, $D_2O$ exchangeable, 1-H); (Found: C, 53.57; H, 5.28; N, 17.95%; $C_{17}H_{19}N_5O_4$.0.25 $CHCl_3$ requires: C, 53.51; H, 5.01; N, 18.09%).

Example 33

9-(4-Hexanoyloxy-3-hydroxymethylbut-1-yl)guanine

To a solution of $N^2$-monomethoxytrityl-9-(4-monomethoxytrityloxy-3-hydroxymethylbut-1-yl)guanine (0.72g, 0.9mmol) in pyridine (4ml) was added hexanoyl chloride (0.38ml, 2.7mmol) and the solution was stirred for 20 minutes. The mixture was precipitated in water (40ml) and the resulting precipitate was stirred in 80% acetic acid (10ml) at 80° for 45 minutes. The solvent was removed and the residue purified by column chromatography on silica gel eluting with chloroform-methanol mixtures (7:1, 5:1) to afford 9-(4-hexanoyloxy-3-hydroxymethylbut-1-yl)guanine (0.12g, 38%), m.p. 179-181°C; νmax 2960, 2930, 1730, 1690, 1630, and 1600 $cm^{-1}$; $\delta_H$ [$(CD_3)_2SO$] 0.84 (3H, t, J 6.9Hz, $CH_3$), 1.24 (4H, m, $CH_3(CH_2)_2$), 1.50 (2H, quintet, J 7.3Hz, $CH_2CH_2CO$), 1.6-1.8 (3H, m, 2'-H and 3'-H), 2.26 (2H, t, J 7.3Hz, $CH_2CO$), 3.40 (2H, t, J 5Hz, $D_2O$ exchange leaves d, $CH_2OH$), 3.9-4.1 (4H, m, 1'-H and $CH_2OCO$), 4.60 (1H, t, J 5.1Hz, $D_2O$ exchangeable, 2-$NH_2$), 7.67 (1H, s, 8-H), and 10.49 (1H, s, $D_2O$ exchangeable, 1-H); (Found: C, 52.63; H, 6.91; N, 18.75%; $C_{16}H_{25}N_5O_4$ requires: C, 54.69; H, 7.17; N, 19.93%).

Example 34

9-(4-Hexadecanoyloxy-3-hydroxymethylbut-1-yl)guanine

To a solution of $N^2$-monomethoxytrityl-9-(4-monomethoxytrityloxy-3-hydroxymethylbut-1-yl)guanine (0.72g, 0.9mmol) in pyridine (4ml) was added hexadecanoyl chloride (0.82ml, 2.7mmol) and the solution was stirred for 30 minutes. The mixture was precipitated in water (40ml) and the resulting precipitate was stirred in 80% acetic acid (8ml) at 80° for 2 hours. The solvent was removed and the residue was purified by column chromatography on silica gel eluting with chloroform-methanol mixtures (10:1, 8:1) to afford 9-(4-hexadecanoyloxy-3-hydroxymethylbut-1-yl)guanine (0.23g, 52%), m.p. 183-191°C; νmax (KBr) 3340, 3160, 2920, 2850, 1740, 1690, and 1605 $cm^{-1}$; $\delta_H$ [$(CD_3)_2SO$] 0.85 (3H, t, J 6.6Hz, $CH_3$), 1.23 (24H, m, $CH_3(CH_2)_{12}$), 1.49 (2H, m, $CH_2CH_2CO$), 1.6-1.8 (3H, m, 2'-H and 3'-H), 2.26 (2H, t, J 7.3Hz, $CH_2CO$), 3.39 (2H, t, J 5Hz, $D_2O$ exchange leaves d, $CH_2OH$), 3.9-4.1 (4H, m, 1'-H and $CH_2OCO$), 4.60 (1H, t, J 5.2Hz, $D_2O$ exchangeable, OH), 6.38 (2H, s, $D_2O$ exchangeable, 2-$NH_2$), 7.67 (1H, s, 8-H), and 10.50 (1H, s, $D_2O$ exchangeable, 1-H); (Found: C, 63.83; H, 9.44; N, 14.05%; $C_{26}H_{45}N_5O_4$ requires: C, 63.51; H, 9.23; N, 14.24%).

Example 35

9-(4-Hydroxy-3-hydroxymethylbut-1-yl)guanine 4'-phosphate diammonium salt

To a solution of cyanoethyl phosphoric acid (4.8mmol) in pyridine (6ml) were added $N^2$-monomethoxytrityl-9-(4-monomethoxytrityloxy-3-hydroxymethylbut-1-yl)guanine (1.28g, 1.6mmol) and dicyclohexylcarbodiimide (1.98g, 9.6mmol) and the solution was stirred for 2 hours. Reaction was quenched by addition of water (1ml) and the solvent was removed. To the residue was added concentrated aqueous ammonia and the mixture was stirred at 60° for 3 hours. The solvent was removed and to the residue was added 80% acetic acid (15ml). The mixture was stirred at 80° for 45 minutes and the solvent was removed. The residue was taken up in water (25ml) and extracted with chloroform (4 x 30ml). The aqueous layer was filtered, concentrated and passed down a column of XAD-4 resin, eluting with aqueous methanol mixtures. Fractions containing product were pooled and the solvent removed. The residue was taken up in a small volume of water and the solution was passed through $C_{18}$-Sep-pak cartridges. Fractions containing product were pooled and the solvent removed to afford 9-(4-hydroxy-3-hydroxymethylbut-1-yl)guanine 4'-phosphate, diammonium salt as a white solid (0.31g, 53%); $\nu$max (KBr) 3150 (broad), 1690, and 1610 $cm^{-1}$; $\delta_H$ [$(CD_3)_2SO/D_2O$] 1.57 (1H, m, 3'-H), 1.70 (2H, m, 2'-H), 3.37 (2H, d, J 4.7Hz, $CH_2OH$), 3.72 (2H, m, $CH_2OP$), 3.99 (2H, t, J 6.9Hz, 1'-H), and 7.68 (1H, s, 8-H).

Example 36

$N^2$-Acetyl-9-(4-hydroxy-3-hydroxymethylbut-1-yl)guanine

To a suspension of 9-(4-hydroxy-3-hydroxymethylbut-1-yl)guanine (0.23g, 0.9mmol) in pyridine (3ml) was added chlorotrimethylsilane (0.25ml, 2.0mmol) and the mixture was stirred for 15 minutes. To this mixture was added acetyl chloride (0.085ml, 1.2mmol) and the mixture was stirred for 30 minutes. Methanol (2ml) was the added and the mixture was stirred for a further 30 minutes. The solvent was removed and the residue purified by column chromatography on silica gel eluting with chloroform-methanol mixtures (4:1, 5:2) to give the title compound as its hydrochloride salt. This was dissolved in methanol and stirred with potassium carbonate. The solution was filtered and the solvent removed. The residue was taken up in water and passed through $C_{18}$-Sep-pak cartridges. Fractions containing product were pooled and the solvent removed to afford $N^2$-acetyl-9-(4-hydroxy-3-hydroxymethylbut-1-yl)guanine (0.11g, 41%), m.p. 143-146°C; $\lambda$max ($H_2O$) 260 (15,100) nm; $\nu$max (KBr) 3420, 3200, 2940, 1685, 1615, and 1560 $cm^{-1}$; $\delta_H$ [$(CD_3)_2SO$] 1.46 (1H, m, 3'-H), 1.77 (2H, q, J 7.1Hz, 2'-H), 2.18 (3H, s, $CH_3$), 3.3-3.5 (4H, m, 2 x 4'-H), 4.13 (2H, t, J 7.4Hz, 1'-H), 4.42 (2H, br.t, J 5Hz, $D_2O$ exchangeable, 2 x OH), 7.98 (1H, s, 8-H), and 11.83 (2H, br, $D_2O$ exchangeable, 1-H and 2-NH).

Example 37

$N^2$-Hexanoyl-9-(4-hydroxy-3-hydroxymethylbut-1-yl)guanine

To a suspension of 9-(4-hydroxy-3-hydroxymethylbut-1-yl)guanine (0.25g, 1.0mmol) in pyridine (5ml) was added chloro-trimethylsilane (0.32ml, 2.5mmol) and the mixture was stirred for 15 minutes. To this mixture was added hexanoyl chloride (0.18ml, 1.3mmol) and the mixture was stirred for 20 minutes. Methanol (2ml) was then added and the mixture was stirred for a further 20 minutes. 1,8-Diazabicyclo-[5.4.O]undec-7-ene (0.57ml, 3.8mmol) and water (0.5ml) were added and the solvent was removed. The residue was purified by column chromatography on silica gel eluting with chloroform-methanol (4:1). Product containing fractions were pooled and the solvent removed. The residue was taken up in water and passed through $C_{18}$-Sep-pak cartridges to afford $N^2$-hexanoyl-9-(4-hydroxy-3-hydroxymethylbut-1-yl)guanine (50mg, 14%), m.p. 86-88°C; $\nu$max (KBr) 3400, 2960, 2940, 1675, 1610, and 1560 $cm^{-1}$; $\delta_H$ [$(CD_3)_2SO$] 0.88 (3H, t, J 6.7Hz, $CH_3$) 1.29 (4H, m, $CH_3(CH_2)_2$), 1.46 (1H, m, 3'-H), 1.60 (2H, m, $CH_2CH_2CO$), 1.77 (2H, q, J 7.1Hz, 2'-H), 2.46 (2H, t, J 7.4Hz, $CH_2CO$) 3.3-3.5 (4H, m, 2 x 4'-H), 4.13 (2H, t, J 7.4Hz, 1'-H), 4.41 (2H, t, J 4.7Hz, $D_2O$ exchangeable, 2 x OH), 7.98 (1H, s, 8-H), 11.65 (1H, br, $D_2O$ exchangeable, NH), and 12.01 (1H, br, $D_2O$ exchangeable, NH); (Found: C, 53.64; H, 7.56; N, 18.95%; $C_{16}H_{25}N_5O_4$ .$0.5H_2O$ requires: C, 53.32; H, 7.27; N, 19.43%).

Example 38

2-Amino-9-(4-hydroxy-3-hydroxymethylbut-1-yl)-6-isopropoxypurine

A solution of 2-amino-6-chloro-9-[2-(2,2-dimethyl-1,3-dioxan-5-yl)ethyl]purine (0.25g, 0.8mmol) in isopropanol (2.5ml) containing sodium isopropoxide (0.5 M) was stirred at 60° for 25 minutes. After cooling, hydrochloric acid (5 M, 0.3ml) and water (0.7ml) were added and the solution was stirred for 15 minutes at room temperature. The solution was neutralised by addition of aqueous sodium bicarbonate and the solvent was removed. The residue was extracted with chloroform-ethanol (2:1) and the solution purified by column chromatography on silica gel eluting with chloroform-methanol (7:1) to afford 2-amino-9-(4-hydroxy-3-hydroxymethylbut-1-yl)-6-isopropoxypurine which was crystallised from chloroform-carbon tetrachloride (0.18g, 76%), m.p. 111.5-113.5°C; $\delta_H$ [$(CD_3)_2SO$] 1.34 (6H, d, J 6.3Hz, $C(CH_3)_2$), 1.45 (1H, m, 3'-H), 1.74 (2H, q, J 7.2Hz, 2'-H), 3.3-3.5 (4H, AB part of ABX, 2 x 4'-H), 4.06 (2H, t, J 7.3Hz, 1'-H), 4.40 (2H, br, 2 x OH), 5.49 (1H, septet, J 6.3Hz, $CH(CH_3)_2$), 6.27 (2H, s, 2-$NH_2$), and 7.83 (1H, s, 8-H).

Example 39

2-Amino-9-(4-hydroxy-3-hydroxymethylbut-1-yl)-6-phenoxypurine

To a solution of phenol (113mg, 1.2mmol) in dry dioxan (2.5ml) was added sodium hydride (60% dispersion in oil; 48mg, 1.2mmol). After evolution of hydrogen ceased, 2-amino-6-chloro-9-[2-(2,2-dimethyl-1,3-dioxan-5-yl)ethyl]purine (0.25g, 0.8mmol) was added and the mixture was stirred at 75° for 3.5 hours. After cooling, water (0.8ml) and hydrochloric acid (5 M, 0.2ml) were added and the solution was stirred for 30 minutes at room temperature. The solution was neutralised by addition of aqueous sodium bicarbonate and the solvent was removed. The residue was extracted with chloroform-ethanol (2:1) and the solution purified by column chromatography on silica gel eluting with chloroform-methanol (9:1) to afford 2-amino-9-(4-hydroxy-3-hydroxymethylbut-1-yl)-6-phenoxypurine (145mg, 55%), m.p. 173-175°C; $\delta_H$ [$(CD_3)_2SO$] 1.48 (1H, m, 3'-H), 1.78 (2H, q, J 7.1Hz, 2'-H), 3.3-3.5 (4H, m, 2 x 4'-H), 4.12 (2H, t, J 7.4Hz, 1'-H), 4.42 (2H, t, J 5.1Hz, $D_2O$ exchangeable, 2 x OH), 6.35 (2H, s, $D_2O$ exchangeable, 2-$NH_2$), 7.2-7.5 (5H, m, $C_6H_5$), and 7.98 (1H, s, 8-H).

Example of pharmaceutical activity

Method 1

Vero (Arican Green Monkey Kidney) cells were grown to confluence in 24 well multidishes, each well being 1.6cm in diameter. The cells were infected with Herpes simplex type 1 virus (HFEM strain) and overlaid with 0.5ml of 0.9% agarose (w/v) in maintenance medium. The test compound, prepared in maintenance medium in concentrations ranging from 100 to 0.3 µg/ml in half-log dilution steps, was added in 0.5ml volume. The virus infected cultures were then incubated at 37°C for 4 days before fixing in 4% formaldehyde solution and staining with carbol fuchsin. The dishes were then examined to find what concentration of test compound caused a 50% reduction in the number of virus plaques formed ($PDD_{50}$ value) and the minimum concentration of test compound which caused cytotoxicity (MTD).

Method 2

MRC-5 cells were infected in suspension with Herpes simplex type 1 virus, strain SC16. The infected cell suspension was dispensed (0.1ml) in 96 well microtitre plates containing the test drugs in maintenance medium in concentrations ranging from 100 to 0.03 µg/ml in half-log dilution steps (0.1ml per well). The plates were then icubated at 37°Cfor 3 days when the virus cytopathic effect (CPE) in the control wells reached 100%. The plates were fixed in 4% formaldehyde solution and stained with carbol fuchsin. The plates were then examined to find what concentration of test compound recuced the virus CPE by 50% ($IC_{50}$). Plates using uninfected cells were run in parallel to determine the minimum concentration of test compound which caused cytotoxicity (MTD).

Compounds were also tested against Herpes simplex type 2 virus (MS strain) in Vero cells using Method 1 and in MRC-5 cells using Method 2. In the latter test, the incubation time was reduced to 24 hours.

Results

| Example No. | PDD$_{50}$ (µg/ml) | | | |
|---|---|---|---|---|
| | Herpes simplex type 1 virus | | Herpes simplex type 2 virus | |
| | HFEM strain in Vero cells | SC16 strain in MRC-5 cells | MS strain in Vero cells | MS strain in MRC-5 cells |
| 4 | 1.3 | 0.9 | 2.3 | 0.6 |
| 12 | 2.2 | 0.7 | | |
| 13 | 1.7 | 0.7 | | |
| 15 | >100 | >100 | 63 | 49 |
| 22 | >100 | >100 | >100 | 83 |
| 23 | >100 | 25 | >100 | 85 |
| 24 | >100 | >100 | >100 | 65 |
| 25 | 1.9 | 1.0 | 1.6 | 0.9 |
| 27 | 13 | 1.5 | 2.8 | 5.7 |
| 31 | 24 | 10 | | |
| 32 | 95 | 3 | | |
| 34 | 16 | 2 | | |

None of the compounds was cytotoxic at concentrations up to 100µg/ml in any of the tests.

Method 3

Compounds were administered by oral gavage (0.2mmoles/kg in 0.1ml of 1% carboxymethyl cellulose) to 20g female Balb/C mice which had been starved for 18 hours. Fifteen minutes later, blood was collected from three mice by cardiac puncture using heparinised syringes. Equal aliquots were pooled and an equal volume of 16% trichloroacetic acid added. Following centrifugation (8,500g) to remove precipitated proteins, the resulting mixture was analysed by high performance liquid chromatography using a $C_{18}$ Nova-Pak cartridge eluted with Buffer A (50mM $NaH_2PO_4$, pH4.6) and Buffer B (10% Buffer A, 10% water, 80% methanol) in a gradient from 1% to 95% Buffer B. 9-(4-Hydroxy-3-hydroxymethylbut-1-yl) guanine was assayed with a Pye-Unicam PU4021 u.v. detector set at 254nm.

Results

**Concentrations of 9-(4-Hydroxy-3-hydroxymethylbut-1-yl) guanine (Example 4) in the Blood of Mice After Oral Administration of Derivatives**

| Administered compound | Concentration of Example 4 in Blood (μg/ml) |
|---|---|
| Example 15 | 0.2 |
| Example 16 | 2.3 |
| Example 17 | 4.8 |
| Example 22 | 2.0 |
| Example 23 | 2.5 |
| Example 30 | 0.3 |
| Example 35 | 1.1 |

## Claims

**Claims for the following Contracting States: CH DE FR GB IT LI NL**

1. A compound of formula (I)

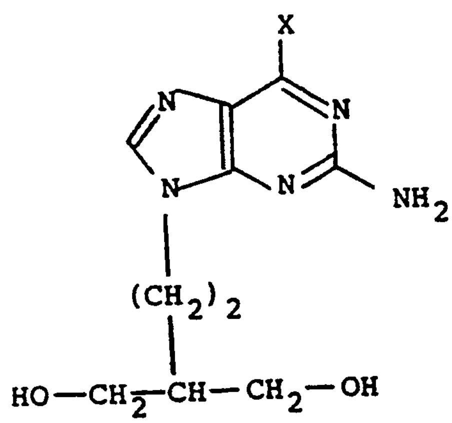

(I)

or a salt, phosphate ester or acyl derivative thereof (as hereinafter defined), in which X represents chlorine, straight or branched chain $C_{1-6}$ alkoxy, phenoxy, phenyl $C_{1-6}$ alkoxy, $-NH_2$, -OH or -SH, an acyl derivative is wherein one or both of the hydrogens in the acyclic -OH groups, and/or one of the hydrogen atoms in the $-NH_2$ group, are replaced by

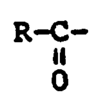

groups, wherein R is hydrogen, $C_{1-18}$ alkyl, phenyl, phenyl $C_{1-6}$ alkyl or imidazolyl;
with the provisos that, i) when X is -OH, the compound of formula (I) is in a purity state of greater than 50% by weight of pure compound with respect to the mono- and di-benzyl ethers thereof; and ii) the compound of formula (I) is other than a compound selected from the group consisting of:

9-[4'-hydroxy-3'-(hydroxymethyl)butyl]guanine cyclic phosphate;
9-[4'-hydroxy-3'-(hydroxymethyl)butyl]guanine cyclic pyrophosphate;
9-[4'-hydroxy-3'-(hydroxymethyl)butyl]-2,6-diaminopurine;
9-[4'-hydroxy 3'-(hydroxymethyl)butyl]-2,6-diaminopurine cyclic phosphosphate;
9-[4'-hydroxy-3'-(hydroxymethyl)butyl]-2,6-diaminopurine cyclic pyrophosphate;
9-[4'-hydroxy-3'-(hydroxymethyl)butyl]-2-amino-6-chloropurine;
9-[4'-hydroxy-3'-(hydroxymethyl)butyl]-2-amino-6-chloropurine cyclic phosphate;
9-[4'-hydroxy-3'-(hydroxymethyl)butyl]-2-amino-6-chloropurine cyclic pyrophosphate;
9-[4'-hydroxy-3'-(hydroxymethyl)butyl]guanine monophosphate monosodium salt;
9-[4'-hydroxy-3'-(hydroxymethyl)butyl]-2,6-diaminopurine monophosphate monosodium salt; and
9-[4'-hydroxy-3'-(hydroxymethyl)butyl]-2-amino-6-chloropurine monophosphate monosodium salt.

**2.** An acyl derivative according to claim 1, in which R is $C_{1-6}$ alkyl, phenyl or benzyl.

**3.** A compound according to claim 1, of formula (II)

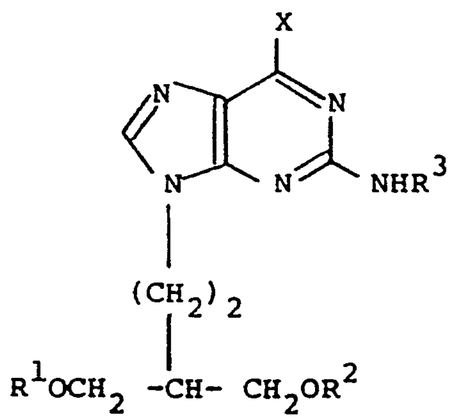

(II)

or a pharmaceutically acceptable salt thereof, in which X is as defined in formula (I), and each of $R^1$, $R^2$ and $R^3$ represents hydrogen or an acyl group of formula

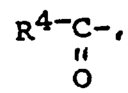

in which $R^4$ is $C_{1-18}$ alkyl or imidazolyl, or $R^1$ or $R^2$ represents a phosphate ester group of formula

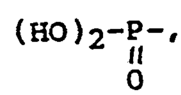

or $R^1$ and $R^2$ together represent a bridging group.

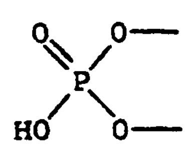

**4.** A compound according to any one of claims 1 to 3 in which X is -OH, or a tautomer thereof.

**5.** A compound according to claim 1, of formula (A)

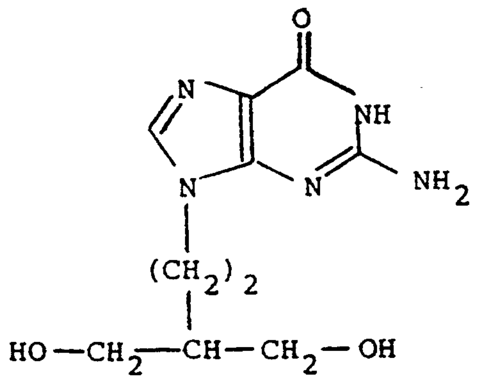

(A)

in a purity state of greater than 60% by weight of pure compound, or a pharmaceutically acceptable salt thereof.

6. A compound according to claim 5, of formula (A), in a purity state of greater than 95% by weight of pure compound, or a pharmaceutically acceptable salt thereof.

7. A compound according to claim 5, of formula A, in the form of an isolated, substantially completely pure compound of formula (A), or a pharmaceutically acceptable salt thereof.

8. A compound according to claim 5, of formula (A), in crystalline form having a melting point of 275-277°C.

9. 9-(4-Hydroxy-3-hydroxymethylbut-1-yl)guanine, sodium salt.

10. A compound according to any one of claims 1 to 9 without the proviso i) of claim 1, for use as an active therapeutic substance.

11. A compound according to claim 10 for use in the treatment of viral infections.

12. A pharmaceutical composition comprising a compound according to any one of claims 1 to 9, without the proviso i) of claim 1, together with a pharmaceutically acceptable carrier or excipient.

13. A process for preparing a compound of formula (A) as defined in claim 6, or a salt thereof, which comprises converting the group X in a compound of formula (III).

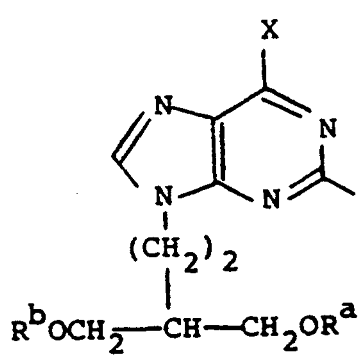

(III)

in which X, excluding -OH, is as defined in claim 1;
$R^a$ and $R^b$, which may be the same or different, are each hydrogen or 0- protecting groups; and Y is chlorine or $-NHR^c$, in which $R^c$ is hydrogen or acyl,

to an -OH group by means of hydrolysis when X is other than $NH_2$, or, when X is $-NH_2$ by means of a deaminase reaction or when Y is chlorine, and X is -OH converting Y to a $-NH_2$ group by reaction with

ammonia under pressure, and subsequently, if desired, converting the compound of formula (A) to a salt thereof by treatment with acid or base.

**14.** A process for preparing a compound according to claim 1, which comprises treating a compound of formula (IV)

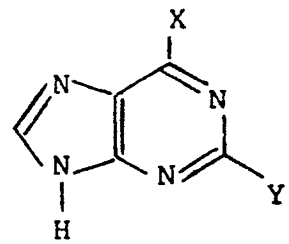

(IV)

in which X is as defined in formula (I) and Y is as defined in formula (III) in claim 13, with a compound of formula (V)

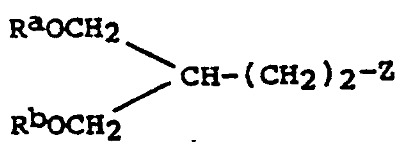

(V)

in which $R^a$ and $R^b$ are as defined in formula (III) in claim 13 and Z is a leaving group.

**15.** A process for preparing an acyl derivative of a compound of formula (I) as defined in claim 1, which comprises acylating an optionally protected compound of formula (I) and, where necessary, deprotecting the resulting product.

**16.** A process for preparing a phosphate ester of a compound of formula (I) according to claim 1 which comprises treating a protected intermediate of the compound of formula (I) in which one of the acyclic -OH groups and the $-NH_2$ group are protected, with cyanoethyl phosphoric acid and subsequently deprotecting the resultant product, by treatment with acid.

**17.** A process for preparing a compound of formula (I) as defined in claim 1, or a salt thereof, which comprises hydrolysing the 1,3-dioxane ring of a compound of formula (VII).

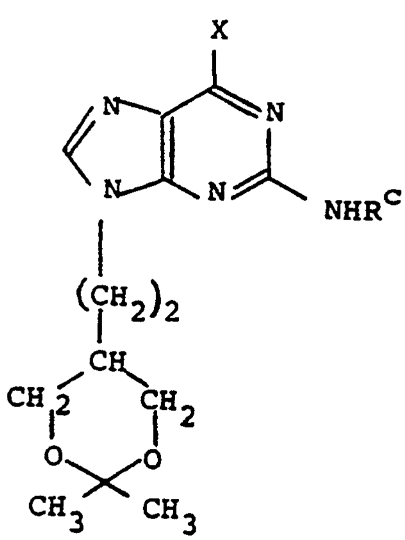

(VII)

in which X is as defined in formula (I) and $R^c$ is as defined in formula (III), provided that $R^c$ is not acyl when X is other than OH, and subsequently, if desired, converting the compound of formula (I) thus formed to a salt by treatment with an acid or base.

**18.** A compound of formula (VII)

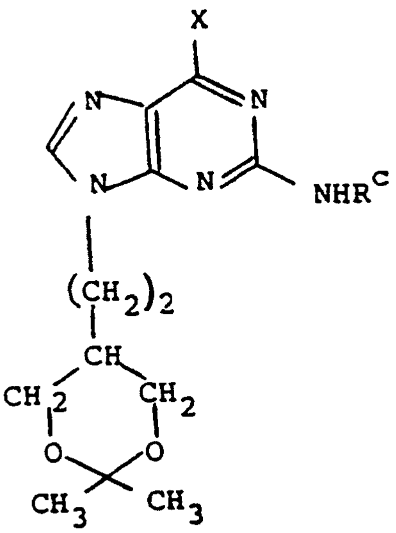

(VII)

in which X is as defined in claim 1 and $R^c$ is hydrogen or acyl.

**19.** A compound of formula:

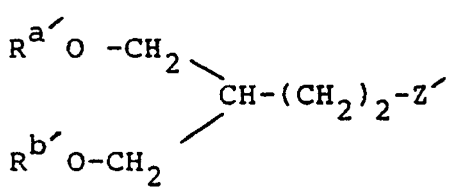

wherein
Z' is hydroxy, chloro, bromo or iodo;
$R^{a'}$ and $R^{b'}$ are

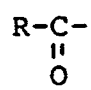

groups, as defined in claim 1; or
$R^{a'}$ and $R^{b'}$ together are

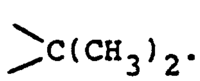

**20.** 5-(2-Hydroxyethyl)-2,2-dimethyl-1,3-dioxan,
5-(2-bromoethyl)-2,2-dimethyl-1,3-dioxan,
2-acetoxymethyl-4-hydroxybut-1-yl acetate, or
2-acetoxymethyl-4-bromobut-1-yl acetate.

**21.** Use of a compound according to any one of claims 1 to 9, without the proviso i) of claim 1, in the manufacture of a medicament for use in the treatment of viral infections.

**22.** Use of a compound according to any one of claims 1 to 9, without the provisos i) and ii) of claim 1, in the manufacture of a medicament for the treatment of herpes simplex type 1 or herpes simplex type 2 viral infections.

**23.** Use of a compound according to any one of claims 1 to 9, without provisos i) and ii) of claim 1, in the manufacture of a medicament for the treatment of varicella-zoster viral infections.

**Claims for the following Contracting States: BE SE**

**1.** A compound of formula (I)

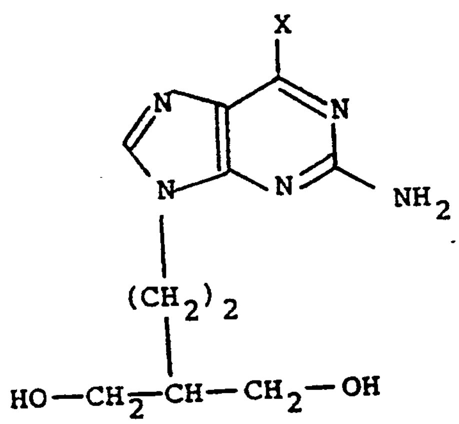

(I)

or a salt, phosphate ester or acyl derivative thereof (as hereinafter defined), in which X represents chlorine, straight or branched chain $C_{1-6}$ alkoxy, phenoxy, phenyl $C_{1-6}$ alkoxy, $-NH_2$, -OH or -SH, an acyl derivative is wherein one or both of the hydrogens in the acyclic -OH groups, and/or one of the hydrogen atoms in the $-NH_2$ group, are replaced by

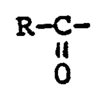

groups, wherein R is hydrogen,
$C_{1-18}$ alkyl, phenyl, phenyl $C_{1-6}$ alkyl or imidazolyl;
with the proviso that, when X is -OH, the compound of formula (I) is in a purity state of greater than 50% by weight of pure compound with respect to the mono- and di-benzyl ethers thereof.

**2.** An acyl derivative according to claim 1, in which R is $C_{1-6}$ alkyl, phenyl or benzyl.

**3.** A compound according to claim 1, of formula (II)

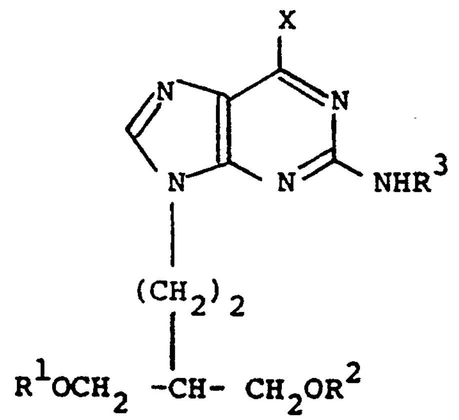

(II)

or a pharmaceutically acceptable salt thereof, in which X is as defined in formula (I), and each of $R^1$, $R^2$ and $R^3$ represents hydrogen or an acyl group of formula

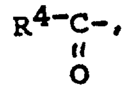

in which $R^4$ is $C_{1-18}$ alkyl or imidazolyl, or $R^1$ or $R^2$ represents a phosphate ester group of formula

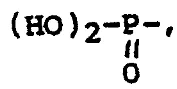

or $R^1$ and $R^2$ together represent a bridging group.

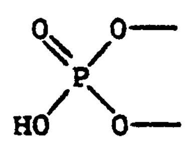

4. A compound according to any one of claims 1 to 3 in which X is -OH, or a tautomer thereof.

5. A compound according to claim 1, of formula (A)

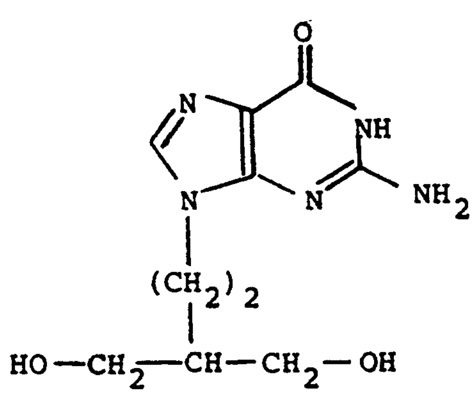

(A)

in a purity state of greater than 60% by weight of pure compound, or a pharmaceutically acceptable salt thereof.

6. A compound according to claim 5, of formula (A), in a purity state of greater than 95% by weight of pure compound, or a pharmaceutically acceptable salt thereof.

7. A compound according to claim 5, of formula A, in the form of an isolated, substantially completely pure compound of formula (A), or a pharmaceutically acceptable salt thereof.

8. A compound according to claim 5, of formula (A), in crystalline form having a melting point of 275-277°C.

9. 9-(4-Hydroxy-3-hydroxymethylbut-1-yl)guanine, sodium salt.

10. A compound according to any one of claims 1 to 9 without the proviso of claim 1, for use as an active therapeutic substance.

11. A compound according to claim 10 for use in the treatment of viral infections.

12. A pharmaceutical composition comprising a compound according to any one of claims 1 to 9, without the proviso of claim 1, together with a pharmaceutically acceptable carrier or excipient.

13. A process for preparing a compound of formula (A) as defined in claim 6, or a salt thereof, which comprises converting the group X in a compound of formula (III).

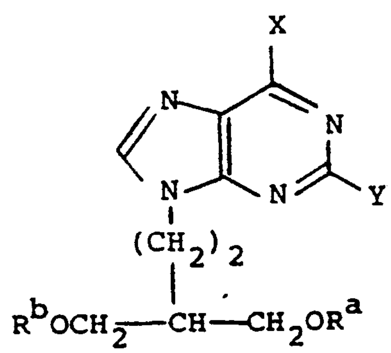

(III)

in which X, excluding -OH, is as defined in claim 1;
$R^a$ and $R^b$, which may be the same or different, are each hydrogen or 0- protecting groups; and Y is chlorine or $-NHR^c$, in which $R^c$ is hydrogen or acyl,

to an -OH group by means of hydrolysis when X is other than $NH_2$, or, when X is $-NH_2$ by means of a deaminase reaction or when Y is chlorine, and X is -OH converting Y to a $-NH_2$ group by reaction with ammonia under pressure, and subsequently, if desired, converting the compound of formula (A) to a salt thereof by treatment with acid or base.

14. A process for preparing a compound according to claim 1, which comprises treating a compound of formula (IV)

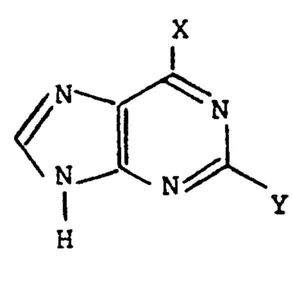

(IV)

in which X is as defined in formula (I) and Y is as defined in formula (III) in claim 13, with a compound

of formula (V)

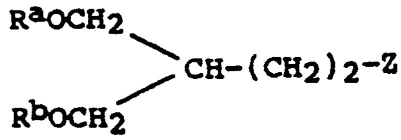

(V)

in which $R^a$ and $R^b$ are as defined in formula (III) in claim 13 and Z is a leaving group.

15. A process for preparing an acyl derivative of a compound of formula (I) as defined in claim 1, which comprises acylating an optionally protected compound of formula (I) and, where necessary, deprotecting the resulting product.

16. A process for preparing a phosphate ester of a compound of formula (I) according to claim 1 which comprises treating a protected intermediate of the compound of formula (I) in which one of the acyclic -OH groups and the $-NH_2$ group are protected, with cyanoethyl phosphoric acid and subsequently deprotecting the resultant product, by treatment with acid.

17. A process for preparing a compound of formula (I) as defined in claim 1, or a salt thereof, which comprises hydrolysing the 1,3-dioxane ring of a compound of formula (VII).

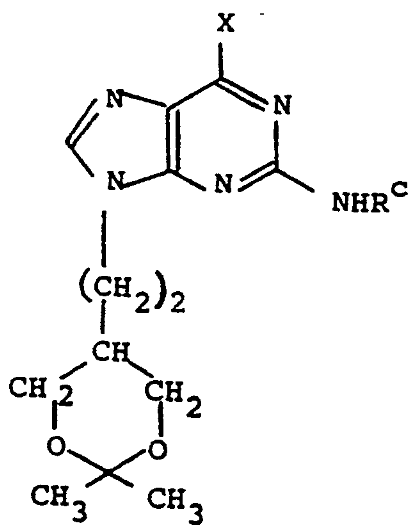

(VII)

in which X is as defined in formula (I) and $R^c$ is as defined in formula (III), provided that $R^c$ is not acyl when X is other than OH, and subsequently, if desired, converting the compound of formula (I) thus formed to a salt by treatment with an acid or base.

18. A compound of formula (VII)

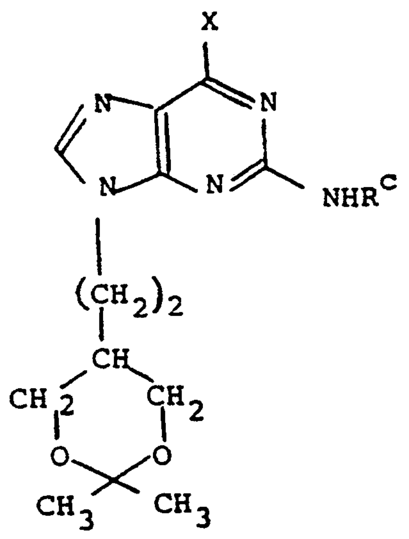

(VII)

in which X is as defined in claim 1 and $R^c$ is hydrogen or acyl.

**19.** A compound of formula:

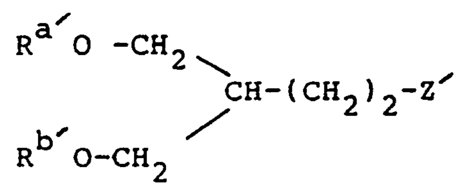

wherein
Z' is hydroxy, chloro, bromo or iodo;
$R^{a'}$ and $R^{b'}$ are

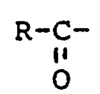

groups, as defined in claim 1; or
$R^{a'}$ and $R^{b'}$ together are

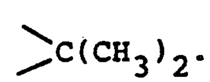

**20.** 5-(2-Hydroxyethyl)-2,2-dimethyl-1,3-dioxan,
5-(2-bromoethyl)-2,2-dimethyl-1,3-dioxan,
2-acetoxymethyl-4-hydroxybut-1-yl acetate, or
2-acetoxymethyl-4-bromobut-1-yl acetate.

**21.** Use of a compound according to any one of claims 1 to 9, without the proviso of claim 1, in the manufacture of a medicament for use in the treatment of viral infections.

**22.** Use of a compound according to any one of claims 1 to 9, without the proviso of claim 1, in the manufacture of a medicament for the treatment of herpes simplex type 1, herpes simplex type 2 or

varicella-zoster viral infections.

**Revendications**

**Revendications pour les Etats contractants suivants: CH DE FR GB IT LI NL**

1. Composé caractérisé par la formule (I) :

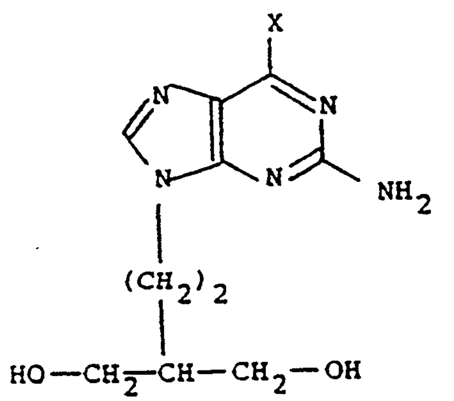

(I)

ou sel, phosphate ou dérivé acyle de celui-ci tel que défini par la suite, dans laquelle X représente un atome de chlore, un groupe alcoxy en $C_{1-6}$ à chaîne droite ou ramifiée, phénoxy, phényl-alcoxy en $C_{1-6}$ , $-NH_2$ , -OH ou -SH , un dérivé acyle étant un dérivé dans lequel un ou deux atomes d'hydrogène dans les groupes -OH acycliques, et/ou un des atomes d'hydrogène du groupe $-NH_2$, sont remplacés par des groupes

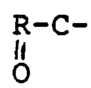 ,

dans lesquels R est un atome d'hydrogène,
un groupe alkyle en $C_{1-18}$ , phényle, phényl-alkyle en $C_{1-6}$ ou imidazolyle ; à condition que (i) lorsque X est -OH, le composé de formule (I) se trouve dans un état de pureté supérieur à 50 % en poids de composé pur par rapport aux éthers mono- et di-benzyliques de celui-ci ;
et (ii) le composé de formule (I) soit autre qu'un composé choisi dans le groupe comprenant les composés suivants :
phosphate cyclique de 9-(4'-hydroxy-3'-(hydroxyméthyl)butyl)guanine,
pyrophosphate cyclique de 9-(4'-hydroxy-3'-(hydroxyméthyl)butyl)guanine,
9-(4'-hydroxy-3'-(hydroxyméthyl)butyl)-2,6-diaminopurine, phosphate cyclique de 9-(4'-hydroxy-3'-(hydroxyméthyl)butyl-2,6-diaminopurine,
pyrophosphate cyclique de 9-(4'-hydroxy-3'-(hydroxyméthyl)butyl)-2,6-diaminopurine,
9-(4'-hydroxy-3'-(hydroxyméthyl)butyl)-2-amino-6-chloropurine,
phosphate cyclique de 9-(4'-hydroxy-3'-(hydroxyméthyl)butyl)-2-amino-6-chloropurine,
pyrophosphate cyclique de 9-(4'-hydroxy-3'-(hydroxyméthyl)butyl-2-amino-6-chloropurine,
sel monosodique de monophosphate de 9-(4'-hydroxy-3'-(hydroxyméthyl)butyl)guanine,
sel monosodique de monophosphate de 9-(4'-hydroxy-3'-(hydroxyméthyl)butyl)-2,6diaminopurine.

2. Dérivé acyle suivant la revendication 1, caractérisé en ce que R est un groupe alkyle en $C_{1-6}$ , phényle ou benzyle.

3. Composé suivant la revendication 1, caractérisé par la formule (II) :

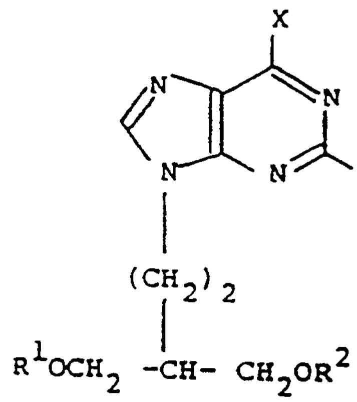

(II)

ou un sel de celui-ci acceptable du point de vue pharmaceutique, dans laquelle X est tel que défini dans la formule (I) et où chacun des $R^1$, $R^2$ et $R^3$ représente un atome d'hydrogène ou un groupe acyle de formule

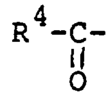

dans laquelle $R^4$ est un groupe alkyle en $C_{1-18}$ ou imidazolyle, ou $R^1$ ou $R^2$ représente un groupe phosphate de formule

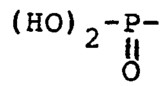

ou bien $R^1$ et $R^2$ représentent ensemble un groupe de pontage

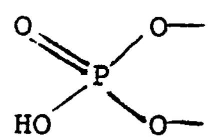

**4.** Composé suivant l'une quelconque des revendications 1 à 3, caractérisé en ce que X est -OH, ou un tautomère de celui-ci.

**5.** Composé suivant la revendication 1, de formule (A) :

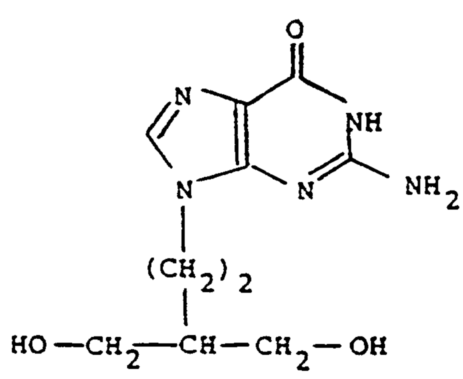

(A)

caractérisé en ce qu'il se trouve dans un état de pureté supérieur à 60 % en poids de composé pur ou

un sel de celui-ci acceptable du point de vue pharmaceutique.

**6.** Composé suivant la revendication 5 de formule (A), caractérisé en ce qu'il se trouve dans un état de pureté supérieur à 95 % en poids de composé pur ou un sel de celui-ci acceptable du point de vue pharmaceutique.

**7.** Composé suivant la revendication 5 de formule (A), caractérisé en ce qu'il est sous forme d'un composé de formule (A) isolé pratiquement complètement pur ou d'un sel de celui-ci acceptable du point de vue pharmaceutique.

**8.** Composé suivant la revendication 5 de formule (A), caractérisé en ce qu'il se trouve sous forme cristalline ayant un point de fusion de 275 - 277° C.

**9.** Composé, caractérisé en ce qu'il s'agit du sel de sodium de la 9-(4-hydroxy-3-hydroxyméthylbut-1-yl)-guanine.

**10.** Composé suivant l'une quelconque des revendications 1 à 9, sans la condition (i) de la revendication 1, caractérisé en ce qu'il est utile en tant que substance thérapeutique active.

**11.** Composé suivant la revendication 10, caractérisé en ce qu'il est utile dans le traitement d'infections virales.

**12.** Composition pharmaceutique, caractérisée en ce qu'elle comprend un composé suivant l'une quelconque des revendications 1 à 9, sansla condition (i) de la revendication 1, avec un support ou excipient acceptable du point de vue pharmaceutique.

**13.** Procédé pour préparer un composé de formule (A) suivant la revendication 6, ou un sel de celui-ci, caractérisé en ce qu'il comprend la conversion du groupe X dans un composé de formule (III) :

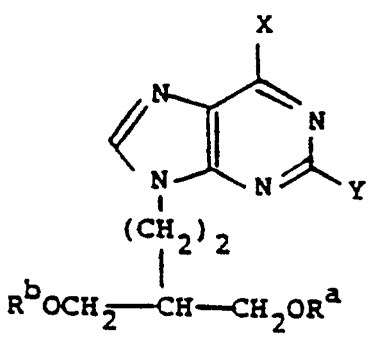

(III)

dans laquelle X, à l'exclusion de -OH est tel que défini dans la revendication 1 ; $R^a$ et $R^b$ qui peuvent être identiques ou différents sont chacun un atome d'hydrogène ou des groupes protégeant l'atome d'oxygène et Y est un atome de chlore ou un groupe $-NHR^c$ dans lequel $R^c$ est un atome d'hydrogène ou un groupe acyle,
en un groupe -OHpar hydrolyse lorsque X est autre que $NH_2$ ou, lorsque X est $-NH_2$ au moyen d'une réaction par une désaminase ou lorsque Y est un atome de chlore et que X est un groupe -OH, la conversion de Y en un groupe $-NH_2$ par réaction avec de l'ammoniac sous pression et ensuite si cela est désiré, la conversion du composé de formule (A) en un sel de celui-ci par traitement avec un acide ou une base.

**14.** Procédé pour préparer un composé suivant la revendication 1, qui comprend le traitement d'un composé de formule (IV) :

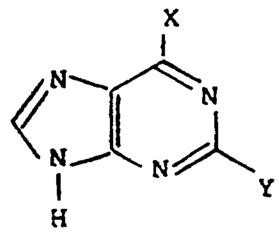

(IV)

dans laquelle X est tel que défini dans la formule (I) et Y est tel que défini dans la formule (III) dans la revendication 13, avec un composé de formule (V) :

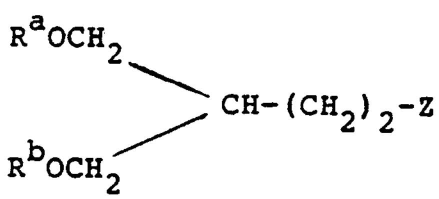

(V)

dans laquelle $R^a$ et $R^b$ sont tels que définis dans la formule (III) dans la revendication 13 et Z est un groupe mobile.

**15.** Procédé pour préparer un dérivé acyle d'un composé de formule (I) suivant la revendication 1, caractérisé en ce qu'il comprend l'acylation d'un composé éventuellement protégé de formule (I), et si nécessaire, la déprotection du produit résultant.

**16.** Procédé pour préparer un phosphate d'un composé de formule (I) suivant la revendication 1, caractérisé en ce qu'il comprend le traitement d'un intermédiaire protégé du composé de formule (I) dans lequel l'un des groupes -OH acycliques et le groupe $-NH_2$ sont protégés, avec de l'acide cyanoéthyle phosphorique et ensuite la déprotection du produit résultant par traitement avec un acide.

**17.** Procédé pour préparer un composé de formule (I) suivant la revendication 1 ou un sel de celui-ci, caractérisé en ce qu'il comprend l'hydrolyse du cycle 1,3-dioxane d'un composé de formule (VII) :

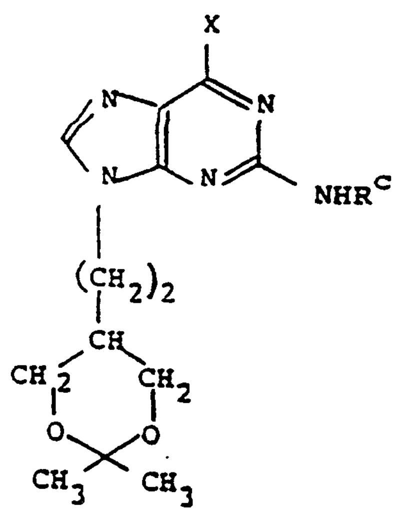

(VII)

dans laquelle X est tel que défini dans la formule (I) et $R^c$ est tel que défini dans la formule (III), à condition que $R^c$ ne soit pas un groupe acyle lorsque X est autre que le groupe -OH et ensuite, si désiré, la conversion du composé de formule (I) ainsi formé en un sel par traitement avec un acide ou une base.

**18.** Composé, caractérisé par la formule (VII) :

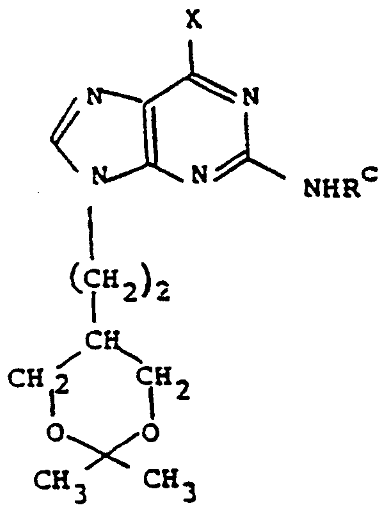

(VII)

dans laquelle X est tel que défini dans la revendication 1 et $R^c$ est un atome d'hydrogène ou un groupe acyle.

**19.** Composé, caractérisé par la formule :

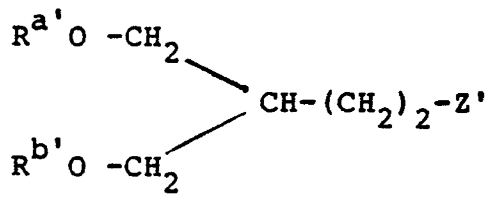

dans laquelle
Z' est un groupe hydroxy, un atome de chlore, de brome ou iode ;
$R^{a'}$ et $R^{b'}$ sont des groupes

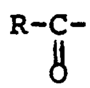 ,

tels que définis dans la revendication 1 ;
$R^{a'}$ et $R^{b'}$ forment ensemble un groupe

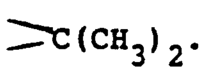

**20.** Composé en ce qu'il s'agit des composés suivants :
5-(2-hydroxyéthyl)-2,2-diméthyl-1,3-dioxane,
5-(2-bromoéthyl)2,2-diméthyl-1,3-dioxane,
acétate de 2-acétoxyméthyl-4-hydroxybut-1-yle, ou
acétate de 2-acétoxyméthyl-4-bromobut-1-yle.

**21.** Utilisation d'un composé suivant l'une quelconque des revendications 1 à 9, sans la condition (i) de la revendication 1, dans la fabrication d'un médicament utile dans le traitement d'infections virales.

**22.** Utilisation d'un composé suivant l'une quelconque des revendications 1 à 9, sans les conditions (i) et (ii) de la revendication 1, dans la fabrication d'un médicament pour le traitement d'infections par les

virus de l'herpès simplex type 1 ou de l'herpès simplex type 2.

**23.** Utilisation d'un composé suivant l'une quelconque des revendications 1 à 9, sans les conditions (i) et (ii) de la revendication 1, dans la fabrication d'un médicament pour le traitement des infections par le virus de varicelle-zoster.

**Revendications pour les Etats contractants suivants: BE SE**

**1.** Composé, caractérisé par la formule (I) :

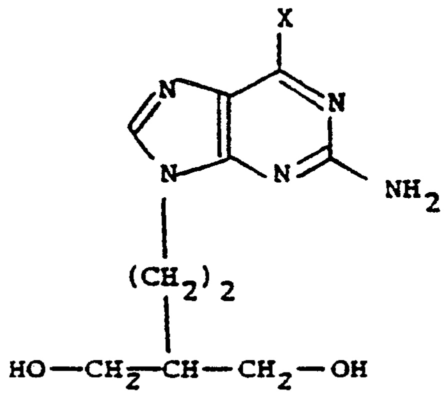

(I)

ou un sel, phosphate ou dérivé acyle de celui-ci tel que défini par la suite, dans laquelle X représente un atome de chlore, un groupe alcoxy en $C_{1-6}$ à chaîne droite ou ramifiée, phénoxy, phényl-alcoxy en $C_{1-6}$ , $NH_2$ , -OH ou -SH, un dérivé acyle étant un dérivé dans lequel un ou les deux atomes d'hydrogène dans les groupes -OH acycliques, et/ou un ou les atomes d'hydrogène dans le groupe $-NH_2$ sont remplacés par des groupes

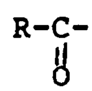

dans lesquels R est un atome d'hydrogène, un groupe alkyle en $C_{1-18}$ , phényle, phényl-alkyle en $C_{1-6}$ ou imidazolyle ; à condition que lorsque X est -OH, le composé de formule (I) se trouve dans un état de pureté supérieur à 50 % en poids de composé pur par rapport aux éthers mono- et dibenzyliques de celui-ci.

**2.** Dérivé acyle suivant la revendication 1, caractérisé en ce que R est un groupe alkyle en $C_{1-6}$ , phényle ou benzyle.

**3.** Composé suivant la revendication 1, caractérisé par la formule (II) :

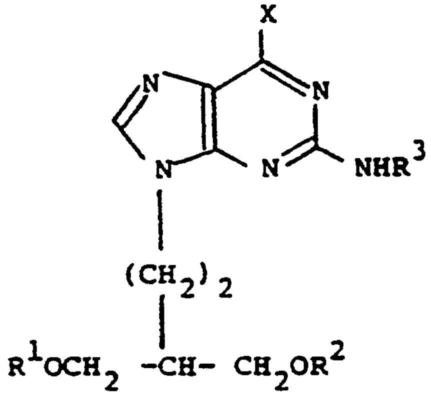

(II)

ou un sel de celui-ci acceptable du point de vue pharmaceutique, dans laquelle X est tel que défini dans la formule (I) et chacun des $R^1$, $R^2$ et $R^3$ représente un atome d'hydrogène ou un groupe acyle de formule

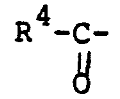

dans laquelle $R^4$ est un groupe alkyle en $C_{1-18}$ ou imidazolyle, ou bien $R^1$ ou $R^2$ représente un groupe phosphate de formule

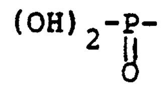

ou bien $R^1$ et $R^2$ représentent ensemble un

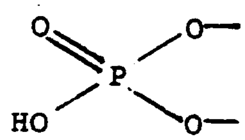

groupe de pontage

4. Composé suivant l'une quelconque des revendications 1 à 3, caractérisé en ce que X est -OH ou un tautomère de celui-ci.

5. Composé suivant la revendication 1 de formule (A) :

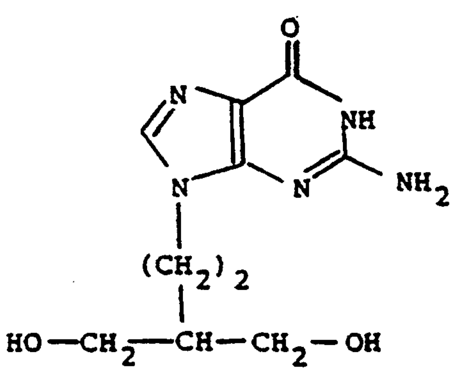

(A)

caractérisé en ce qu'il se trouve dans un état de pureté supérieur à 60 % en poids du composé pur, ou un sel de celui-ci acceptable du point de vue pharmaceutique.

6. Composé suivant la revendication 5 de formule (A), caractérisé en ce qu'il se trouve dans un état de pureté supérieur à 95 % en poids du composé pur ou un sel de celui-ci acceptable du point de vue pharmaceutique.

7. Composé suivant la revendication 5 de formule (A), caractérisé en ce qu'il se trouve sous la forme d'un composé de formule (A) isolé, pratiquement complètement pur ou d'un sel de celui-ci acceptable du point de vue pharmaceutique.

8. Composé suivant la revendication 5 de formule (A), caractérisé en ce qu'il se trouve sous forme cristalline ayant un point de fusion de 275 - 277 °C.

**9.** Composé , caractérisé en ce qu'il s'agit du sel de sodium de 9-(4-hydroxy-3-hydroxyméthylbut-1-yl)-guanine.

**10.** Composé suivant l'une quelconque des revendications 1 à 9, sans la condition de la revendication 1, caractérisé en ce qu'il est utile en tant que substance thérapeutique active.

**11.** Composé suivant la revendication 10, caractérisé en ce qu'il est utile dans le traitement d'infections virales.

**12.** Composition pharmaceutique, caractérisée en ce qu'elle comprend un composé suivant l'une quelconque des revendications 1 à 9, sans la condition de la revendication 1, avec un support ou excipient acceptable du point de vue pharmaceutique.

**13.** Procédé pour préparer un composé de formule (A) suivant la revendication 6 ou un sel de celui-ci, caractérisé en ce qu'il comprend la conversion du groupe X dans un composé de formule (III) :

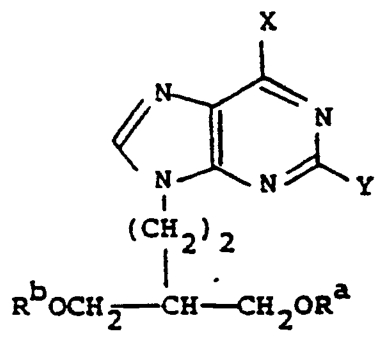

(III

dans laquelle X à l'exclusion de -OH est tel que défini dans la revendication 1, $R^a$ et $R^b$ qui peuvent être identiques ou différents sont chacun un atome d'hydrogène ou des groupes protégeant l'atome d'oxygène et Y est un atome de chlore ou un groupe $-NHR^c$ dans lequel $R^c$ est un atome d'hydrogène ou un groupe acyle,
en un groupe -OH par hydrolyse lorsque X est autre que $NH_2$ ou, lorsque X est $-NH_2$ au moyen d'une réaction par une désaminase ou lorsque Y est un atome de chlore et lorsque X est -OH, la conversion de Y en un groupe $-NH_2$ par réaction avec de l'ammoniac sous pression, et ensuite, si désiré, la conversion du composé de formule (A) en un sel de celui-ci par traitement avec un acide ou une base.

**14.** Procédé pour préparer un composé suivant la revendication 1, caractérisé en ce qu'il comprend le traitement d'un composé de formule (IV) :

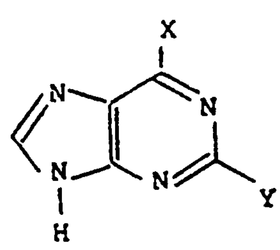

(IV)

dans laquelle X est tel que défini dans la formule (I) et Y est tel que défini dans la formule (III) dans la revendication 13, avec un composé de formule (V) :

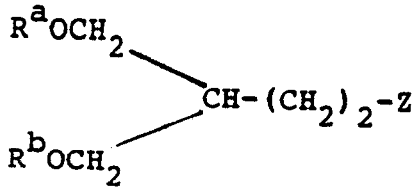

(V)

dans laquelle $R^a$ et $R^b$ sont tels que définis dans la formule (III) dans la revendication 13 et Z est un groupe mobile.

**15.** Procédé pour préparer un dérivé acyle d'un composé de formule (I) suivant la revendication 1, caractérisé en ce qu'il comprend l'acylation d'un composé éventuellement protégé de formule (I) et, si nécessaire, la déprotection du produit résultant.

**16.** Procédé pour préparer un phosphate d'un composé de formule (I) suivant la revendication 1, caractérisé en ce qu'il comprend le traitement d'un intermédiaire protégé du composé de formule (I) dans laquelle l'un des groupes -OH acycliques et le groupe $-NH_2$ sont protégés, avec de l'acide cyanoéthylphosphorique et ensuite la déprotection du produit résultant par traitement avec un acide.

**17.** Procédé pour préparer un composé de formule (I) suivant la revendication 1 ou un sel de celui-ci, caractérisé en ce qu'il comprend l'hydrolyse du cycle 1,3-dioxane d'un composé de formule (VII) :

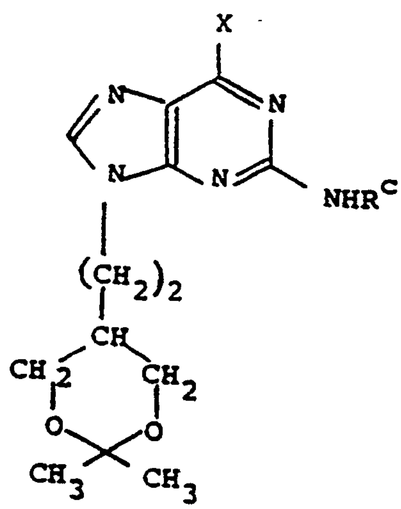

(VII)

dans laquelle X est tel que défini dans la formule (I) et $R^c$ est tel que défini dans la formule (III), à condition que $R^c$ ne soit pas un groupe acyle lorsque X est autre que -OH et ensuite, si désiré, la conversion du composé de formule (I) ainsi formé en un sel par traitement avec un acide ou une base.

**18.** Composé, caractérisé par la formule (VII) :

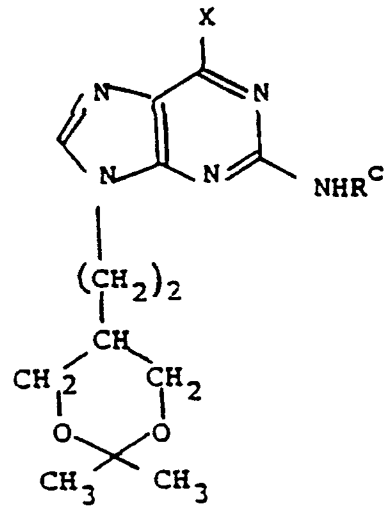

(VII)

dans laquelle X est tel que défini dans la revendication 1 et $R^c$ est un atome d'hydrogène ou un groupe acyle.

**19.** Composé, caractérisé par la formule :

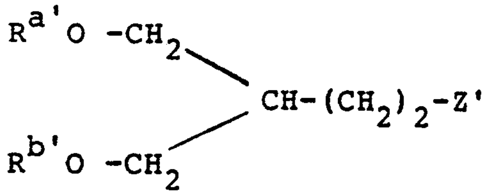

dans laquelle Z' est un groupe hydroxy, un atome de chlore, de brome ou d'iode ;
$R^{a'}$ et $R^{b'}$ sont des groupes

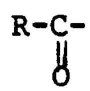 ,

tels que définis dans la revendication 1 ; ou
$R^{a'}$ et $R^{b'}$ forment ensemble un groupe

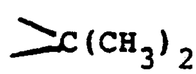 .

**20.** Composé caractérisé en ce qu'il s'agit des composés suivants :
5-(2-hydroxyéthyl)-2,2-diméthyl-1,3-dioxane,
5-(2-bromoéthyl)-2,2-diméthyl-1,3-dioxane,
acétate de 2-acétoxyméthyl-4-hydroxybut-1-yle, ou
acétate de 2-acétoxyméthyl-4-bromobut-1-yle.

**21.** Utilisation d'un composé suivant l'une quelconque des revendications 1 à 9, sans la condition de la revendication 1, dans la fabrication d'un médicament utile dans le traitement d'infections virales.

**22.** Utilisation d'un composé suivant l'une quelconque des revendications 1 à 9, sans la condition de la revendication 1, dans la fabrication d'un médicament pour le traitement d'infections par les virus de

l'herpès simplex type 1, l'herpès simplex type 2 ou de la varicelle-zoster.

**Patentansprüche**
**Patentansprüche für folgende Vertragsstaaten: CH DE FR GB IT LI NL**

1. Verbindung der Formel (I)

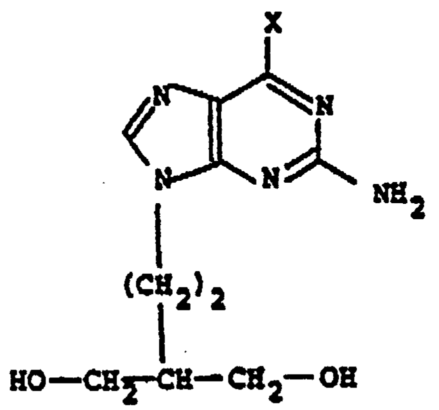

(I)

oder dessen Salz, Phosphatester oder Acylderivat, wie nachstehend definiert, in der X ein Chloratom, einen geradkettigen oder verzweigtkettigen $C_{1-6}$-Alkoxyrest, eine Phenoxygruppe, einen Phenyl-$C_{1-6}$-alkoxyrest, eine $NH_2$-, OH- oder SH-Gruppe bedeutet, und im Falle des Acylderivats eines oder beide der Wasserstoffatome der acyclischen OH-Gruppen und/oder eines der Wasserstoffatome der $NH_2$-Gruppe durch einen Rest

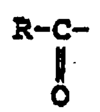

ersetzt sind, wobei R ein Wasserstoffatom,
einen $C_{1-18}$-Alkylrest, eine Phenylgruppe, einen Phenyl-$C_{1-6}$-alkylrest oder eine Imidazolylgruppe bedeutet, mit den Maßgaben, daß i) wenn X eine OH-Gruppe ist, die Verbindung der Formel (I) in einer Reinheit von größer als 50 Gew.-% der reinen Verbindung bezogen auf deren Mono- und Dibenzylether ist und ii) die Verbindung der Formel (I) nicht
9-[4'-Hydroxy-3'-(hydroxymethyl)butyl]guanidin-cyclisches-Phosphat,
9-[4'-Hydroxy-3'-(hydroxymethyl)butyl]guanidin-cyclisches-Pyrophosphat,
9-[4'-Hydroxy-3'-(hydroxymethyl)butyl]-2,6-diaminopurin,
9-[4'-Hydroxy-3'-(hydroxymethyl)butyl]-2,6-diaminopurin-cyclisches-Phosphat,
9-[4'-Hydroxy-3'-(hydroxymethyl)butyl]-2,6-diaminopurin-cyclisches-Pyrophosphat,
9-[4'-Hydroxy-3'-(hydroxmethyl)butyl]-2-amino-6-chlorpurin,
9-[4'-Hydroxy-3'-(hydroxymethyl)butyl]-2-amino-6-chlorpurin-cyclisches-Phosphat,
9-[4'-Hydroxy-3'-(hydroxymethyl)butyl]-2-amino-6-chlorpurin-cyclisches-Pyrophosphat,
9-[4'-Hydroxy-3'-(hydroxymethyl)butyl]guanidinmono-phosphat-Mononatriumsalz,
9-[4'-Hydroxy-3'-(hydroxymethyl)-butyl]-2,6-diaminopurin-monophosphat- Mononatriumsalz und
9-[4'-Hydroxy-3'-(hydroxymethyl)butyl]-2-amino-6-chlorpurin-monophosphat-Mononatriumsalz
ist.

2. Acylderivat nach Anspruch 1, wobei R ein $C_{1-6}$-Alkylrest, eine Phenyl- oder Benzylgruppe ist.

3. Verbindung der Formel (II) nach Anspruch 1

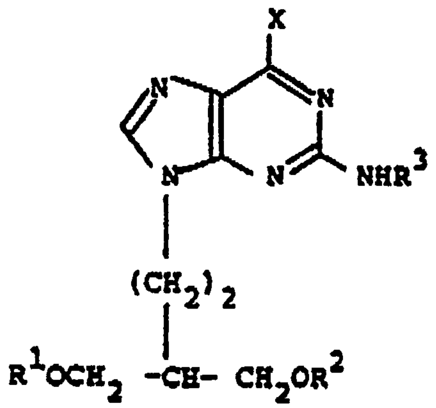

(II)

oder dessen pharmazeutisch verträgliches Salz, in der X wie in Formel (I) definiert ist und jeder der Reste $R^1$, $R^2$ und $R^3$ ein Wasserstoffatom oder einen Acylrest der Formel

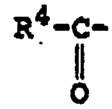

darstellt, wobei $R^4$ ein $C_{1-18}$-Alkylrest oder eine Imidazolylgruppe ist, oder $R^1$ oder $R^2$ einen Phosphatesterrest der Formel

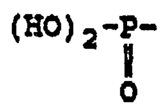

darstellen oder $R^1$ und $R^2$ zusammen eine brückenbildende Gruppe

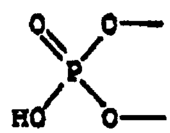

darstellen.

4. Verbindung nach einem der Ansprüche 1 bis 3, wobei X eine OH-Gruppe ist oder dessen Tautomer.

5. Verbindung der Formel (A) nach Anspruch 1

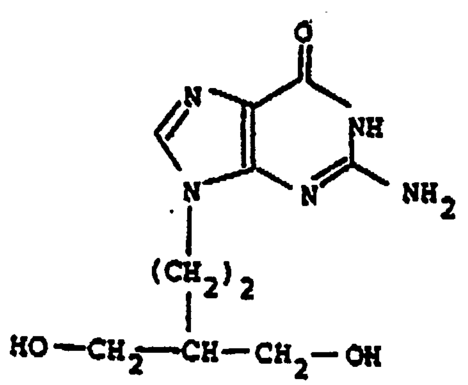

(A)

in einer Reinheit von größer als 60 Gew.-% der reinen Verbindung oder dessen pharmazeutisch

verträgliches Salz.

6. Verbindung der Formel (A) nach Anspruch 5 in einer Reinheit von größer als 95 Gew.-% der reinen Verbindung oder dessen pharmazeutisch verträgliches Salz.

7. Verbindung der Formel (A) nach Anspruch 5 in Form einer isolierten, im wesentlichen völlig reinen Verbindung der Formel (A) oder dessen pharmazeutisch verträgliches Salz.

8. Verbindung der Formel (A) nach Anspruch 5 in kristalliner Form mit einem Schmelzpunkt von 275 bis 277 °C.

9. 9-(4-Hydroxy-3-hydroxymethylbut-1-yl)guanidin Natriumsalz.

10. Verbindung nach einem der Ansprüche 1 bis 9 ohne die Maßgabe i) des Anspruches 1 zur Verwendung als therapeutische Wirksubstanz.

11. Verbindung nach Anspruch 10 zur Verwendung bei der Behandlung von viralen Infektionen.

12. Arzneimittel, das eine Verbindung nach einem der Ansprüche 1 bis 9 ohne die Maßgabe i) des Anspruches 1 zusammen mit einem pharmazeutisch verträglichen Träger oder Exzipiens umfaßt.

13. Verfahren zur Herstellung einer Verbindung der Formel (A) wie in Anspruch 6 definiert oder dessen Salzes, das die Umwandlung der Gruppe X in der Verbindung der Formel (III)

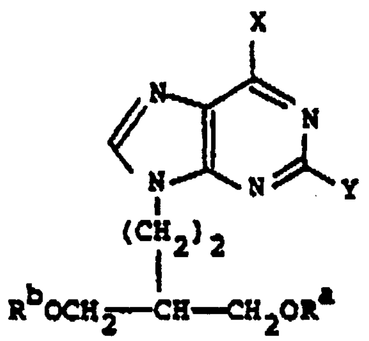

(III)

in der X mit Ausnahme der OH-Gruppe wie in Anspruch 1 definiert ist, $R^a$ und $R^b$ gleich oder verschieden sind und Wasserstoffatome oder O-Schutzgruppen bedeuten und Y ein Chloratom oder einen Rest -NHR$^c$ bedeutet, in dem $R^c$ ein Wasserstoffatom oder ein Acylrest ist, mittels Hydrolyse in eine OH-Gruppe, falls X keine $NH_2$-Gruppe ist oder wenn X eine $NH_2$-Gruppe ist durch eine Desaminierungsreaktion, oder wenn Y ein Chloratom und X eine OH-Gruppe ist, Umwandlung von Y in eine $NH_2$-Gruppe durch Umsetzung mit Ammoniak unter Druck und nachfolgend, falls gewünscht, Umwandlung der Verbindung der Formel (A) in dessen Salz durch Behandlung mit Säure oder Base umfaßt.

14. Verfahren zur Herstellung einer Verbindung nach Anspruch 1, das die Behandlung einer Verbindung der Formel (IV)

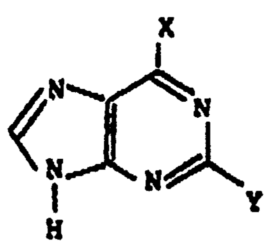

(IV)

in der X wie in Formel (I) definiert ist und Y wie in Formel (III) von Anspruch 13 definiert ist, mit einer

Verbindung der Formel (V)

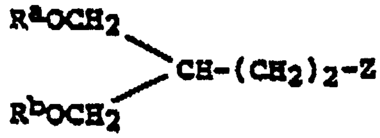

(V)

in der $R^a$ und $R^b$ wie in Formel (III) von Anspruch 13 definiert sind und Z eine Austrittsgruppe ist, umfaßt.

**15.** Verfahren zur Herstellung eines Acylderivats der Verbindung der Formel (I), wie in Anspruch 1 definiert, das die Acylierung einer gegebenenfalls geschützten Verbindung der Formel (I) und, falle nötig, das Entschützen des erhaltenen Produkts umfaßt.

**16.** Verfahren zur Herstellung eines Phosphatesters der Verbindung der Formel (I) nach Anspruch 1, das die Behandlung einer geschützten Zwischenstufe der Verbindung der Formel (I), in der eine der acyclischen OH-Gruppen und der $NH_2$-Gruppen geschützt sind, mit Cyanoethylphosphorsäure und danach Entschützen des erhaltenen Produkts durch Behandlung mit Säure umfaßt.

**17.** Verfahren zur Herstellung einer Verbindung der Formel (I) wie in Anspruch 1 definiert, oder dessen Salz, das die Hydrolyse des 1,3-Dioxanrings der Verbindung der Formel (VII)

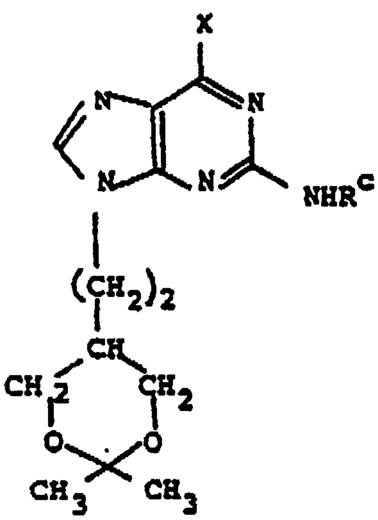

(VII)

wobei X wie in Formel (I) definiert ist und $R^c$ wie in Formel (III) definiert ist, mit der Maßgabe, daß $R^c$ kein Acylrest ist, wenn X keine OH-Gruppe ist und nachfolgend, falls gewünscht, Umwandlung der so hergestellten Verbindung der Formel (I) in ein Salz durch Behandlung mit einer Säure oder Base umfaßt.

**18.** Verbindung der Formel (VII)

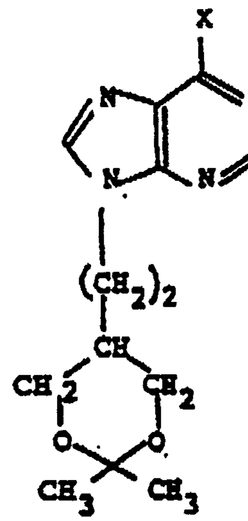

(VII)

wobei X wie in Anspruch 1 definiert ist und $R^c$ ein Wasserstoffatom oder ein Acylrest ist.

**19.** Verbindung der Formel

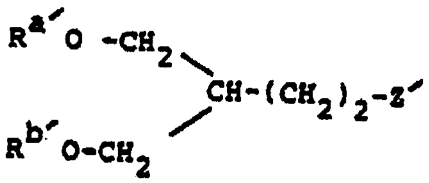

wobei Z' eine Hydroxygruppe, ein Chlor-, Brom- oder Iodatom ist, $R^{a'}$ und $R^{b'}$ Reste

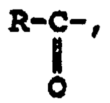

wie in Anspruch 1 definiert, sind, oder
$R^{a'}$ und $R^{b'}$ zusammen eine $>C(CH_3)_2$-Gruppe bilden.

**20.** 5-(2-Hydroxyethyl)-2,2-dimethyl-1,3-dioxan,
5-(2-Bromethyl)-2,2-dimethyl-1,3-dioxan,
2-Acetoxymethyl-4-hydroxybut-1-yl-acetat oder
2-Acetoxymethyl-4-brombut-1-yl-acetat.

**21.** Verwendung einer Verbindung nach einem der Ansprüche 1 bis 9 ohne die Maßgabe i) von Anspruch 1 zur Herstellung eines Arzneimittels zur Verwendung bei der Behandlung von viralen Infektionen.

**22.** Verwendung einer Verbindung nach einem der Ansprüche 1 bis 9 ohne die Maßgaben i) und ii) von Anspruch 1 bei der Herstellung eines Arzneimittels zur Behandlung von Herpes simplex Typ 1 oder Herpes simplex Typ 2 virale Infektionen.

**23.** Verwendung einer Verbindung nach einem der Ansprüche 1 bis 9 ohne die Maßgaben i) und ii) von Anspruch 1 bei der Herstellung eines Arzneimittels zur Behandlung von Varicella-Zoster virale Infektionen.

**Patentansprüche für folgende Vertragsstaaten: BE SE**

1. Verbindung der Formel (I)

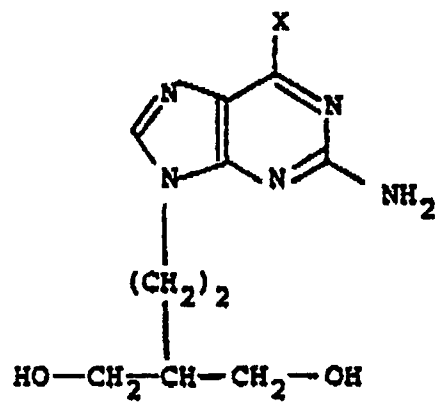

(I)

oder dessen Salz, Phosphatester oder Acylderivat, wie nachstehend definiert, in der X ein Chloratom, einen geradkettigen oder verzweigtkettigen $C_{1-6}$-Alkoxyrest, eine Phenoxygruppe, einen Phenyl-$C_{1-6}$-alkoxyrest, eine $NH_2$-, OH- oder SH-Gruppe bedeutet, und im Falle des Acylderivats eines oder beide der Wasserstoffatome der acyclischen OH-Gruppen und/oder eines der Wasserstoffatome der $NH_2$-Gruppe durch einen Rest

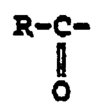

ersetzt sind, wobei R ein Wasserstoffatom,
einen $C_{1-18}$-Alkylrest, eine Phenylgruppe, einen Phenyl-$C_{1-6}$-alkylrest oder eine Imidazolylgruppe bedeutet, mit den Maßgaben, daß, wenn X eine OH-Gruppe ist, die Verbindung der Formel (I) In einer Reinheit von größer als 50 Gew.-% der reinen Verbindung bezogen auf deren Mono- und Dibenzylether ist.

2. Acylderivat nach Anspruch 1, wobei R ein $C_{1-6}$-Alkylrest, eine Phenyl- oder Benzylgruppe ist.

3. Verbindung der Formel (II) nach Anspruch 1

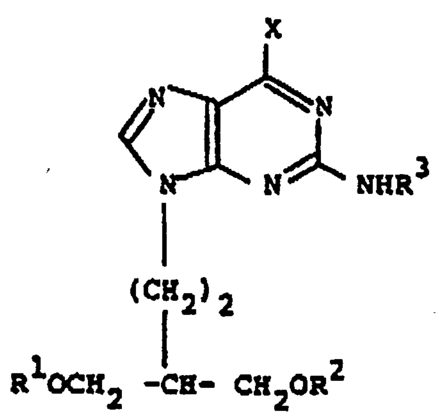

(II)

oder dessen pharmazeutisch verträgliches Salz, in der X wie in Formel (I) definiert ist und jeder der Reste $R^1$, $R^2$ und $R^3$ ein Wasserstoffatom oder einen Acylrest der Formel

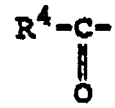

darstellt, wobei $R^4$ ein $C_{1-18}$-Alkylrest oder eine Imidazolylgruppe ist, oder $R^1$ oder $R^2$ einen Phosphatesterrest der Formel

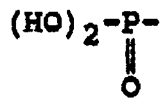

darstellen oder $R^1$ und $R^2$ zusammen eine brückenbildende Gruppe

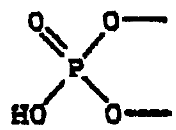

darstellen.

4. Verbindung nach einem der Ansprüche 1 bis 3, wobei X eine OH-Gruppe ist oder dessen Tautomer.

5. Verbindung der Formel (A) nach Anspruch 1

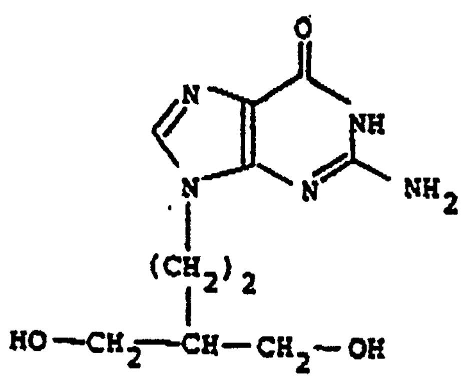

(A)

in einer Reinheit von größer als 60 Gew.-% der reinen Verbindung oder dessen pharmazeutisch verträgliches Salz.

6. Verbindung der Formel (A) nach Anspruch 5 in einer Reinheit von größer als 95 Gew.-% der reinen Verbindung oder dessen pharmazeutisch verträgliches Salz.

7. Verbindung der Formel (A) nach Anspruch 5 in Form einer isolierten, im wesentlichen völlig reinen Verbindung der Formel (A) oder dessen pharmazeutisch verträgliches Salz.

8. Verbindung der Formel (A) nach Anspruch 5 in kristalliner Form mit einem Schmelzpunkt von 275 bis 277 °C.

9. 9-(4-Hydroxy-3-hydroxymethylbut-1-yl)guanidin-Natriumsalz.

10. Verbindung nach einem der Ansprüche 1 bis 9 ohne die Maßgabe des Anspruches 1 zur Verwendung als therapeutische Wirksubstanz.

**11.** Verbindung nach Anspruch 10 zur Verwendung bei der Behandlung von viralen Infektionen.

**12.** Arzneimittel, das eine Verbindung nach einem der Ansprüche 1 bis 9 ohne die Maßgabe des Anspruches 1 zusammen mit einem pharmazeutisch verträglichen Träger oder Exzipiens umfaßt.

**13.** Verfahren zur Herstellung einer Verbindung der Formel (A) wie in Anspruch 6 definiert oder dessen Salzes, das die Umwandlung der Gruppe X in der Verbindung der Formel (III)

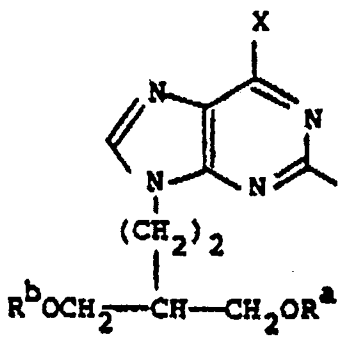

(III)

in der X mit Ausnahme der OH-Gruppe wie in Anspruch 1 definiert ist, $R^a$ und $R^b$ gleich oder verschieden sind und Wasserstoffatome oder O-Schutzgruppen bedeuten und Y ein Chloratom oder einen Rest $-NHR^c$ bedeutet, in dem $R^c$ ein Wasserstoffatom oder ein Acylrest ist, mittels Hydrolyse in eine OH-Gruppe, falls X keine $NH_2$-Gruppe ist oder wenn X eine $NH_2$-Gruppe ist durch eine Desaminierungsreaktion, oder wenn Y ein Chloratom und X eine OH-Gruppe ist, Umwandlung von Y in eine $NH_2$-Gruppe durch Umsetzung mit Ammoniak unter Druck und nachfolgend, falls gewünscht, Umwandlung der Verbindung der Formel (A) in dessen Salz durch Behandlung mit Säure oder Base umfaßt.

**14.** Verfahren zur Herstellung einer Verbindung nach Anspruch 1, das die Behandlung einer Verbindung der Formel (IV)

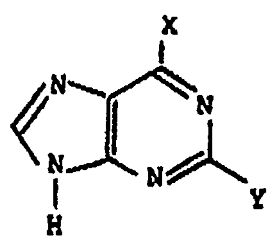

(IV)

in der X wie in Formel (I) definiert ist und Y wie in Formel (III) von Anspruch 13 definiert ist, mit einer Verbindung der Formel (V)

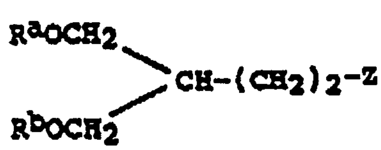

(V)

in der $R^a$ und $R^b$ wie in Formel (III) von Anspruch 13 definiert sind und Z eine Austrittsgruppe ist, umfaßt.

**15.** Verfahren zur Herstellung eines Acylderivats der Verbindung der Formel (I), wie in Anspruch 1 definiert, das die Acylierung einer gegebenenfalls geschützten Verbindung der Formel (I) und, falls nötig, das Entschützen des erhaltenen Produkts umfaßt.

**16.** Verfahren zur Herstellung eines Phosphatesters der Verbindung der Formel (I) nach Anspruch 1, das die Behandlung einer geschützten Zwischenstufe der Verbindung der Formel (I), in der eine der acyclischen OH-Gruppen und der $NH_2$-Gruppen geschützt sind, mit Cyanoethylphosphorsäure und danach Entschützen des erhaltenen Produkts durch Behandlung mit Säure umfaßt.

**17.** Verfahren zur Herstellung einer Verbindung der Formel (I) wie in Anspruch 1 definiert, oder dessen Salz, das die Hydrolyse des 1,3-Dioxanrings der Verbindung der Formel (VII)

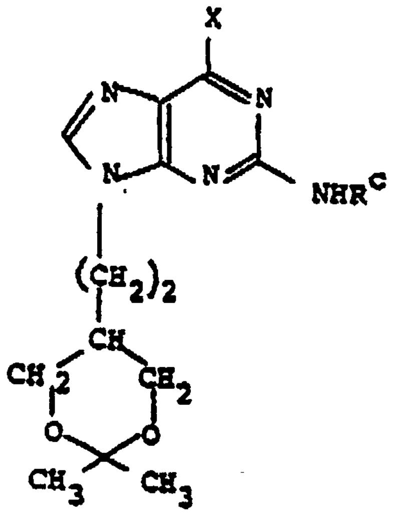

(VII)

wobei X wie in Formel (I) definiert ist und $R^c$ wie in Formel (III) definiert ist, mit der Maßgabe, daß $R^c$ kein Acylrest ist, wenn X keine OH-Gruppe ist und nachfolgend, falls gewünscht, Umwandlung der so hergestellten Verbindung der Formel (I) in ein Salz durch Behandlung mit einer Säure oder Base umfaßt.

**18.** Verbindung der Formel (VII)

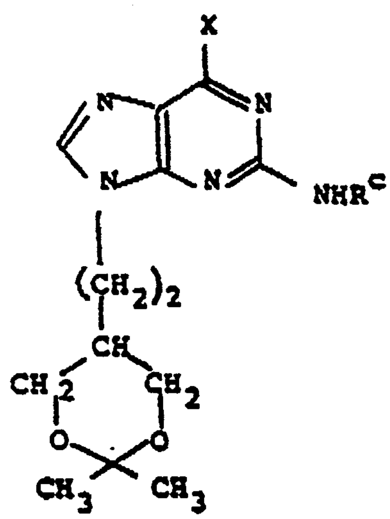

(VII)

wobei X wie in Anspruch 1 definiert ist und $R^c$ ein Wasserstoffatom oder ein Acylrest ist.

**19.** Verbindung der Formel

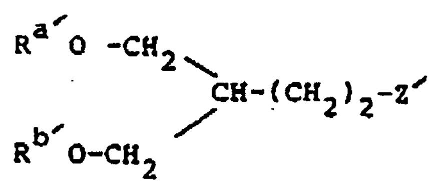

wobei Z' eine Hydroxygruppe, ein Chlor-, Brom- oder lodatom ist, $R^{a'}$ und $R^{b'}$ Reste

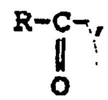

wie in Anspruch 1 definiert, sind, oder
$R^{a'}$ und $R^{b'}$ zusammen eine $>C(CH_3)_2$-Gruppe bilden.

**20.** 5-(2-Hydroxyethyl)-2,2-dimethyl-1,3-dioxan,
5-(2-Bromethyl)-2,2-dimethyl-1,3-dioxan,
2-Acetoxymethyl-4-hydroxybut-1-yl-acetat oder
2-Acetoxymethyl-4-brombut-1-yl-acetat.

**21.** Verwendung einer Verbindung nach einem der Ansprüche 1 bis 9 ohne die Maßgabe von Anspruch 1 zur Herstellung eines Arzneimittels zur Verwendung bel der Behandlung von viralen Infektionen.

**22.** Verwendung einer Verbindung nach einem der Ansprüche 1 bis 9 ohne die Maßgabe von Anspruch 1 bei der Herstellung eines Arzneimittels zur Behandlung von Herpes simplex Typ 1, Herpes simplex Typ 2 oder von Varicella-Zoster virale Infektionen.